# EUROPEAN PATENT APPLICATION

(11) **EP 4 011 388 A1**
(43) Date of publication of application: **15.06.2022**
(21) Application number: 21213209.6
(22) Date of filing: 08.12.2021
(51) Int. Cl.: A61K 38/20, C07K 14/55, A61P 37/00, A61P 7/00, A61P 3/00

(54) **THERAPEUTICALLY ACTIVE ALDESLEUKIN HIGHLY STABLE IN LIQUID PHARMACEUTICAL COMPOSITIONS**

(30) Priority: 09.12.2020 US 202017116722
(71) Applicant: Amcyte Pharma, Inc., Cambridge, MA 02140 (US)
(72) Inventor: RODRIGUEZ, Juan, Manuel, Buenos Aires (AR); VEGA, Julio, César, Buenos Aires (AR)
(74) Representative: HGF

(57) **Abstract**

The disclosure relates to a liquid pharmaceutical composition of aldesleukin/SDS aggregates and its use in the treatment of auto-immune disease, inflammatory disorders, gene therapies and cancer. A method for preparing said composition is also described.

SEQUENCE LISTING

<110>  Amcyte Pharma, Inc

<120>  THERAPEUTICALLY ACTIVE ALDESLEUKIN HIGHLY STABLE IN LIQUID
       PHARMACEUTICAL COMPOSITIONS

<130>  P335194EP

<150>  17/116,722
<151>  2020-12-09

<160>  1

<170>  PatentIn version 3.5

<210>  1
<211>  132
<212>  PRT
<213>  Artificial Sequence

<220>
<223>  Description of Artificial Sequence: Synthetic polypeptide

<400>  1

Pro Thr Ser Ser Ser Thr Lys Lys Thr Gln Leu Gln Leu Glu His Leu
1               5                   10                  15

Leu Leu Asp Leu Gln Met Ile Leu Asn Gly Ile Asn Asn Tyr Lys Asn
            20                  25                  30

Pro Lys Leu Thr Arg Met Leu Thr Phe Lys Phe Tyr Met Pro Lys Lys
        35                  40                  45

Ala Thr Glu Leu Lys His Leu Gln Cys Leu Glu Glu Glu Leu Lys Pro
    50                  55                  60

Leu Glu Glu Val Leu Asn Leu Ala Gln Ser Lys Asn Phe His Leu Arg
65                  70                  75                  80

Pro Arg Asp Leu Ile Ser Asn Ile Asn Val Ile Val Leu Glu Leu Lys
                85                  90                  95

Gly Ser Glu Thr Thr Phe Met Cys Glu Tyr Ala Asp Glu Thr Ala Thr
            100                 105                 110

Ile Val Glu Phe Leu Asn Arg Trp Ile Thr Phe Ser Gln Ser Ile Ile
        115                 120                 125

Ser Thr Leu Thr
    130

## Description

### FIELD

The disclosure relates to new pharmaceutical compositions comprising aldesleukin/SDS aggregates stable in solution and their pharmaceutical uses.

### BACKGROUND

Interleukin 2 (IL-2) is a key cytokine regulating survival, proliferation and differentiation of T cells and NK cells (1). Discovery of these biological activities led to approval for clinical application of IL-2 in cancer immunotherapy for metastatic renal carcinoma in 1994 and for metastatic melanoma in 1998. Afterwards, IL-2 emerged as a reagent to drive *in vitro* expansion of T lymphocytes extracted from a tumor (tumor infiltrating lymphocytes, TILs) of a patient, that were then transfer back to the patient in order to avoid future cancer development (2). Recently, IL-2 has been also used to expand T cells transduced with antigen-specific T cells receptors (3), or transduced with chimeric antigen receptors (CARs) for cancer treatments (4). A new field of IL-2 medical applications recently emerged as a consequence of the discovery that IL-2 have dual functions in the immune response: a) the well-known activity as activator of the inflammatory response and b) as a down regulator of the inflammatory response inducing the expansion of T regulatory cells (CD4⁺Foxp3⁺Treg cells) (5). This seemingly paradoxical duality can be explain by the existence of two kind of IL-2 receptors with high or low affinities and their particular cellular distribution (5). This IL-2 duality resulted in opposing therapies as follows: a) use of IL-2 in high doses to stimulate the immune response like in the original treatments of cancer, and b) use of IL-2 in low doses to depress excessive or aberrant immune response like in the treatment of autoimmune or inflammatory disorders.

Most (if not all) of the current clinical applications of IL-2 are carried out using a recombinant mutated version of natural IL-2 named aldesleukin (des-ala-2ser125) (PROLEUKIN^{™}). US patent 4,604,377 teaches how to prepared a pharmaceutical composition of recombinant IL-2 (including aldesleukin) consistent in a sterile, stable lyophilized formulation in which the recombinant IL-2 is admixed with a water soluble carrier such as mannitol that provides bulk, and sufficient amount of sodium dodecyl sulfate to ensure the solubility of the recombinant IL-2 in water. The formulation is suitable for reconstitution in aqueous injections for parenteral administration stable and well tolerated in human patients. On the other hand, a detailed description of the process by which the PROLEUKIN^{™} product is prepared has been more recently disclosed in the European patent EP 1,688,146 B.

### SUMMARY

Accordingly, certain embodiments of the present disclosure provide methods and compositions concerning the isolation and effective stimulation of cells as well as methods for the use of these cells in the treatment and/or prevention of immune-mediated diseases, cancer, viral infections, or infections mediated by pathogens, neurological disorders and the like.

In certain embodiments, a method to prepare aldesleukin highly stable in liquid pharmaceutical compositions for parenteral administration to a patient in need of an IL-2 immunotherapy, comprising, in general, the following steps: a) Fermentation of an *E.coli* strain transfected with an expression vector engineered to highly express the aldesleukin gene, b) Bacterial disruption, c) Collection of Inclusion bodies containing aldesleukin aggregates, d) Dissolution of the aldesleukin aggregates using SDS detergent, e) Oxidation with an oxidizing compound e.g. cupric chloride, f) Ceramic hydroxyapatite chromatography, g) Dilution with an organic nitrile, e.g. acetonitrile, h) C4 column HPLC chromatography, i) Diafiltration and j) Filter sterilization.

In certain embodiments, a composition comprises aldesleukin in a pharmaceutical suitable aqueous vehicle, for parenteral administration to a patient in need of IL-2 immunotherapy, in which the aldesleukin has been prepared using the production method of the disclosure that ensures that the aldesleukin is stable in a pharmaceutical liquid composition for at least a year. With the composition of aldesleukin of the disclosure it is possible to prepare a pharmaceutical product consistent in one or more pre-filled syringes, each one containing a dose suitable for a given treatment, packaged in a box. This product would not need any manipulation previous to the injection into the patient, avoiding for example microbial or other possible contaminations. On the other hand, the stable liquid aldesleukin preparation of the disclosure could be advantageously introduced and homogeneously distributed in parenteral solutions commonly used in medicine for infusion, transfusion or clinical nutrition. Lately, treatments consisting in a given drug plus aldesleukin has been developed (e.g. CEPLENE^{™} (histamine dihydrochloride) is administered in conjunction with low dose of proleukin for maintenance of first remission in patients with Acute Myeloid Leukemia). For such treatments, the stable liquid aldesleukin preparation of the disclosure may serve as the base to elaborate a unique combined preparation or a combined kit. Proleukin has been also used to induce *in vitro* proliferation of susceptible cells to be used in cell therapy procedures (5). For these procedure, the stable liquid aldesleukin of the disclosure, formulated in low concentrations, can be very advantageous in order to avoid manipulations that can result in microbial contamination and also in order to obtain a rapid and homogeneous distribution of aldesleukin in the cell culture. These are only few examples, however a person in the art, can easily imagine other applications of the stable liquid aldesleukin preparation of the disclosure. In fact, IL-2 preparations are useful for stimulation of NK, T cells modified T cells, modified NK cells, tumor infiltrating lymphocytes (TIL), and all other cells expressing IL-2 receptors in their membranes and may be find applications as a drug in different medical treatments. In certain embodiments, the aldesleukin is used as a drug for medical treatments involving cells expressing IL-2 receptors in their membranes, comprising NK cells, T cells, dendritic cells, nonlymphoid cells, cell lines, transformed cells or combinations thereof.

In certain embodiments, a pharmaceutical composition comprises IL-2, *e.g.* SEQ ID NO: 1, SEQ ID NO: 2, in a range from about 0.001 mg to about 3 mg/mL, SDS 0.0001mg/mL to about 10 mg/mL, a non-ionic osmolytes, a pH 7-8 phosphate buffer or combinations thereof. In certain embodiments, a pharmaceutical composition consists of IL-2 in a range from about 0.001 mg to 3 mg/mL, sodium dodecyl sulfate (SDS) in a range of about 0.0001mg/ml to about 10 mg/mL, a non-ionic osmolytes and a pH 7-8 phosphate buffer. In certain embodiments, an osmolyte comprises mannitol, sorbitol, xylitol or a pH 7-8 phosphate buffer. In certain embodiments composition may comprise an IL-2 protein, *e.g.* SEQ ID NO: 1, plus a buffer, an antioxidant such as ascorbic acid, a low molecular weight polypeptide(such as those having less than 10 amino acids), a protein, amino acids, carbohydrates such as glucose, sucrose, or dextrins, chelating agent such as EDTA, glutathione, and/or other stabilizers, excipients, and/or preservatives. The composition may be formulated as a liquid or a lyophilizate. Further examples of components that may be employed in pharmaceutical formulations are presented in Remington's Pharmaceutical Sciences, 23rd Ed., Mack Publishing Company, Easton, Pa., (2020) and others as described herein.

In certain embodiments, the methods comprise administering a pharmaceutical composition comprising the IL-2, *e.g.* SEQ ID NO: 1, SEQ ID NO: 2 or the combination thereof, to the subject. In some embodiments, the subject is a subject in need thereof.

In certain embodiments, the subject in need thereof is suffering from an inflammatory disorder. In some embodiments, the inflammatory disorder comprises inflammation, autoimmune disease, atopic diseases, paraneoplastic autoimmune diseases, cartilage inflammation, arthritis, rheumatoid arthritis (e.g. active), juvenile arthritis, juvenile rheumatoid arthritis, pauciarticular juvenile rheumatoid arthritis, polyarticular juvenile rheumatoid arthritis, systemic onset juvenile rheumatoid arthritis, juvenile ankylosing spondylitis, juvenile enteropathic arthritis, juvenile reactive arthritis, juvenile Reiter's Syndrome, SEA Syndrome (Seronegativity, Enthesopathy, Arthropathy Syndrome), juvenile dermatomyositis, juvenile psoriatic arthritis, juvenile scleroderma, juvenile systemic lupus erythematosus, juvenile vasculitis, pauciarticular rheumatoid arthritis, polyarticular rheumatoid arthritis, systemic onset rheumatoid arthritis, ankylosing spondylitis, enteropathic arthritis, reactive arthritis, Reiter's Syndrome, SEA Syndrome (Seronegativity, Enthesopathy, Arthropathy Syndrome), dermatomyositis, psoriatic arthritis, scleroderma, vasculitis, myolitis, polymyolitis, dermatomyolitis, polyarteritis nodossa, Wegener's granulomatosis, arteritis, ploymyalgia rheumatica, sarcoidosis, sclerosis, primary biliary sclerosis, sclerosing cholangitis, Sjogren's syndrome, psoriasis, plaque psoriasis, guttate psoriasis, inverse psoriasis, pustular psoriasis, erythrodermic psoriasis, dermatitis, atopic dermatitis, dermatitis herpetiformis, Behcet's disease, including but not limited to the effects on the skin, alopecia, alopecia areata, alopecia totalis, atherosclerosis, lupus, Still's disease, Systemic Lupus Erythematosus(SLE) (e.g. active), myasthenia gravis, inflammatory bowel disease (IBD), Crohn's disease, ulcerative colitis, celiac disease, multiple sclerosis(MS), asthma, COPD, rhinosinusitis, rhinosinusitis with polyps, eosinophilic esophogitis, eosinophilic bronchitis, Guillain-Barredisease, Type I diabetes mellitus, thyroiditis (e.g., Graves' disease),Addison's disease, Raynaud's phenomenon, autoimmune hepatitis, graft versus host disease, steroid refractory chronic graft versus host disease, transplantation rejection (e.g. kidney, lung, heart, skin, and the like), kidney damage, hepatitis C-induced vasculitis, spontaneous loss of pregnancy, alopecia, vitiligo, focal segmental glomerulosclerosis (FSGS), Minimal Change Disease, Membranous Nephropathy, ANCA Associated Glomerulonephropathy, Membranoproliferative Glomerulonephritis, IgA Nephropathy, lupus nephritis, and the like.

In certain embodiments, the immune disorder is inflammation, graft versus host disease, transplant rejection, or an autoimmune disorder. In certain embodiments, the immune disorder is graft versus host disease (GVHD). In specific aspects, the GVHD is chronic GVHD. In certain embodiments, the immune disorder is transplant rejection. In certain embodiments, the transplant is an organ transplant, bone marrow or other cell transplant, composite tissue transplant, or a skin graft. In certain embodiments, the immune disorder is multiple sclerosis, inflammatory bowel disease, rheumatoid arthritis, type I diabetes, systemic lupus erythrematosus, contact hypersensitivity, asthma or Sjogren's syndrome. In certain embodiments, the immune disorder is cancer.

In certain embodiments, a method of treating a subject at risk of or is suffering from an immune disorder comprises obtaining an isolated population of T cells, and culturing the isolated population of T cells in the presence of a pharmaceutical composition comprising IL-2, *e.g.* SEQ ID NO: 1, for a period of time sufficient to induce proliferation of a population of T cells; and wherein, the T cells are adoptively transferred to the subject. In certain embodiments, the T cells are regulatory T (Treg) cells. Regulatory T (Tregs) cells are T lymphocytes having immunosuppressive activity. Natural Tregs are characterized as CD4⁺CD25⁺Foxp3⁺ cells. Humans and mice presenting a genetic deficit in Tregs develop multiple T-cell mediated organ-specific autoimmune diseases. A Treg quantitative or qualitative defect has been described in many human autoimmune diseases, including systemic lupus erythematosus (SLE), Type 1 Diabetes, Multiple Sclerosis, uveitis and myositis. Conversely, addition/restoration of Treg induces clinical improvements in most animal models of these diseases.

Tregs also play a major role in the control of inflammatory diseases, although their mode of action in such disease is not well understood. In fact, in most inflammatory diseases, Treg, depletion exacerbates disease while Treg addition decreases it. This is for example shown in the context of atherosclerosis. Although this disease is not primarily an inflammatory disease, its development involves an inflammatory component/loop. In apolipoprotein E (ApoE) deficient mice that spontaneously develop atherosclerosis, Treg depletion significantly aggravated the plaque formation, while injection of polyclonal Tregs significantly improved the disease. Most Tregs are CD4⁺ cells, although there also a rare population of CD8⁺Foxp3⁺ T lymphocytes with a suppressive activity.

In certain embodiments, a method of inducing proliferation of regulatory T cells in vivo, comprises administering to a subject in need thereof, a therapeutically effective amount of aldesleukin wherein the regulatory T cells increase as compared to a control, inducing proliferation of regulatory T cells in vivo. In certain embodiments, the aldesleukin is comprised in a pharmaceutical composition. In certain embodiments, the aldesleukin comprises SEQ ID NO: 1, SEQ ID NO: 2, or variants thereof. In certain embodiments, the aldesleukin remains stable in the pharmaceutical liquid composition for at least a year as measured by an in vitro biological activity assay. In certain embodiments, the aldesleukin comprises a stable aldesleukin/SDS aggregate distribution in the liquid composition at least for a year in a range of about 4 - 18 nm with a peak at about 8 nm as measured by dynamic light scattering. In certain embodiments, the aldesleukin has a stable therapeutic activity, at least for a year in the liquid composition, as measured in an animal model of human cancer.

In certain embodiments, a method of regulating an immune response associated with a disease or disorder in a subject in need thereof, comprising: administering to a subject in need thereof, a pharmaceutical composition comprising a therapeutically effective amount of aldesleukin wherein the regulatory T cells increase as compared to a control, inducing proliferation of regulatory T cells in vivo. In certain embodiments, the aldesleukin remains stable in the pharmaceutical liquid composition for at least a year as measured by an in vitro biological activity assay. In certain embodiments, the aldesleukin comprises a stable aldesleukin/SDS aggregate distribution in the liquid composition at least for a year in a range of about 4 - 18 nm with a peak at about 8 nm as measured by dynamic light scattering. In certain embodiments, the aldesleukin has a stable therapeutic activity, at least for a year in the liquid composition, as measured in an animal model of human cancer. In certain embodiments, the disease or disorder comprises: an autoimmune disease, cancer, inflammation, a viral infection, a bacterial infection, a neurodegenerative disorder or combinations thereof.

In certain embodiments, a method of inducing proliferation of a population of T cells, comprises obtaining an isolated population of T cells, and culturing the isolated population of T cells in the presence of a pharmaceutical composition comprising IL-2, *e.g.* SEQ ID NO: 1, for a period of time sufficient to induce proliferation of a population of T cells. In certain embodiments, the T cells are regulatory T (Treg) cells.

In certain embodiments, an *in vitro* method of producing a stimulated population of T cells, comprises obtaining an isolated population of T cells, and culturing the isolated population of T cells in the presence a pharmaceutical composition comprising IL-2, *e.g.* SEQ ID NO: 1, SEQ ID NO: 2 or the combination thereof, for a period of time sufficient to produce a stimulated population of T cells.

In certain embodiments, a method of treating an autoimmune disease or disorder in a subject in need thereof, comprises: administering to a subject in need thereof, a pharmaceutical composition comprising a therapeutically effective amount of aldesleukin wherein the regulatory T cells increase as compared to a control, inducing proliferation of regulatory T cells in vivo. In certain embodiments, the aldesleukin remains stable in the pharmaceutical liquid composition for at least a year as measured by an in vitro biological activity assay. In certain embodiments, the aldesleukin comprises a stable aldesleukin/SDS aggregate distribution in the liquid composition at least for a year in a range of about 4 - 18 nm with a peak at about 8 nm as measured by dynamic light scattering. In certain embodiments, the aldesleukin has a stable therapeutic activity, at least for a year in the liquid composition, as measured in an animal model of human cancer.

In certain embodiments, a method of restoring T-cell mediated immune responses in a subject in need thereof comprising administering a therapeutically effective amount of (i) a pharmaceutical composition comprising IL-2 in a range from about 0.001 mg to 3 mg/mL, sodium dodecyl sulfate (SDS) from about 0.0001 mg/ml to about 10 mg/mL, a non-ionic osmolytes(s), or combinations thereof (b) an expression vector encoding a therapeutic protein; (ii) one or more mobilization factors, thereby restoring T-cell mediated immune responses in the subject in need thereof. In certain embodiments, the IL-2 comprises SEQ ID NO: 1, SEQ ID NO: 2, a combination thereof, or variants thereof. In certain embodiments, the osmolytes comprise mannitol, sorbitol, xylitol or a pH 7-8 phosphate buffer.

In certain embodiments, a method of treating a subject suffering from an immune deficiency disease comprising administering to the subject:(i) an expression vector encoding SEQ ID NO: 1; and/or, (ii) a cell comprising an expression vector encoding SEQ ID NO: 1; and/or, (iii) a pharmaceutical composition comprising IL-2 in a range from about 0.001 mg to 3 mg/mL, sodium dodecyl sulfate (SDS) from about 0.0001 mg/ml to about 10 mg/mL, a non-ionic osmolytes(s), or combinations thereof.

In certain embodiments, a method of treating a subject in need of immunotherapy, comprising: administering to the subject: (i) an expression vector encoding SEQ ID NO: 1; and/or, (ii) a cell comprising an expression vector encoding SEQ ID NO: 1, thereby treating the subject. In certain embodiments, the subject is suffering from cancer, immunodeficiency, immunosuppression, viral infections, an immune disorder or combinations thereof. In certain embodiments, the immune disorder, inflammation, graft versus host disease (GVHD), transplant rejection, or an autoimmune disorder. In certain embodiments, the immune disorder is multiple sclerosis, inflammatory bowel disease, rheumatoid arthritis, type I diabetes, systemic lupus erythrematosus, contact hypersensitivity, asthma or Sjogren's syndrome. In certain embodiments, the method further comprises administering IL-2.

In certain embodiments, the cells comprise: autologous cells, allogeneic cells, haplotype matched cells, haplotype mismatched cells, haplo-identical cells, xenogeneic cells, cell lines or combinations thereof. In certain embodiments, the cells comprise stem cells, cord blood cells, adult stem cells, mesenchymal stem cells, mesenchymal stromal cells, induced pluripotent stem cells, autologous stem cells, bone marrow cells, hematopoietic cells, hematopoietic stem cells, somatic cells, germ line cells, differentiated cells, somatic stem cells, embryonic stem cells or combinations thereof.

The IL-2 molecules herein include IL-2 variants of the present disclosure comprising an amino acid sequence that has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the IL-2 amino acid sequence (SEQ ID NO: 1). These include IL-2 variants that comprise an amino acid sequence having an N88R mutation that has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the wild-type IL-2 amino acid sequence (i.e. SEQ ID NO: 1). Embodiments also include IL-2 variants that preferentially stimulate Treg cells and comprise an amino acid sequence having N88R and C125S mutations that has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, or at least 98% sequence identity to the IL-2 amino acid sequence (SEQ ID NO: 1). Embodiments also include IL-2 variants that preferentially stimulate Treg cells and comprise an amino acid sequence having at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to the IL-2 amino acid sequence (SEQ ID NO: 1).

Any compositions or methods provided herein can be combined with one or more of any of the other compositions and methods provided herein.

### BRIEF DESCRIPTION OF THE FIGURES

FIG. 1: Amino acid sequence of the recombinant human interleukin 2 (Aldesleukin) (SEQ ID NO: 1).
FIG. 2: Comparison of the aldesleukin/SDS particle size range, as measured by dynamic light scattering, between PROLEUKIN^{™} and the aldesleukin prepared by the method of this disclosure.
FIG. 3: Aldesleukin/SDS particle size range of the aldesleukin prepared by the method of this disclosure after 0, 6 or 12 month storage at 0-8°C.

### DETAILED DESCRIPTION

Embodiments of the disclosure are directed to a highly stable IL-2 in a liquid formulation and methods of producing the stable IL-2.

### Definitions

The terminology used herein is for the purpose of describing particular embodiments only and is not intended to be limiting of the invention. Unless specifically defined otherwise, all technical and scientific terms used herein shall be taken to have the same meaning as commonly understood by one of ordinary skill in the art (e.g., in cell culture, molecular genetics, and biochemistry).

As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising."

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value or range. Alternatively, particularly with respect to biological systems or processes, the term can mean within an order of magnitude within 5-fold, and also within 2-fold, of a value. Where particular values are described in the application and claims, unless otherwise stated the term "about" meaning within an acceptable error range for the particular value should be assumed.

A "biological medium" as used herein, is any type of medium that is used to grow, culture and maintain organs, tissues, cells etc., *in vitro.* A biological medium also encompasses any biocompatible agent, any pharmaceutical excipient, pharmaceutically and physiologically acceptable fluids such as water, physiological saline, balanced salt solutions, aqueous dextrose, glycerol or the like as a vehicle, tissue or organ culture media, any agent that can be administered *in vivo* to a subject, any agent that can be used in assays or for diluting or maintaining a biological sample, e.g. nucleic acids, peptides etc.

As used herein, "cancer" and "cancerous" refer to or describe the physiological condition in mammals that is typically characterized by unregulated cell growth. Included in this definition are benign and malignant cancers as well as dormant tumors or micrometastatses. Examples of cancer include but are not limited to, carcinoma, lymphoma, blastoma, sarcoma, and leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

As used herein, the term "chemokine" refers to soluble factors (e.g., cytokines) that have the ability to selectively induce chemotaxis and activation of leukocytes. They also trigger processes of angiogenesis, inflammation, wound healing, and tumorigenesis. Example chemokines include IL-8, a human homolog of murine keratinocyte chemoattractant (KC).

The term "chimeric antigen receptor" or "CAR" as used herein refers to an antigen-binding domain that is fused to an intracellular signaling domain capable of activating or stimulating an immune cell, and in certain embodiments, the CAR also comprises a transmembrane domain. In certain embodiments the CAR's extracellular antigen-binding domain is composed of a single chain variable fragment (scFv) derived from fusing the variable heavy and light regions of a murine or humanized monoclonal antibody. Alternatively, scFvs may be used that are derived from Fab's (instead of from an antibody, e.g., obtained from Fab libraries). In various embodiments, the scFv is fused to the transmembrane domain and then to the intracellular signaling domain. "First-generation" CARs include those that solely provide CD3ζ signals upon antigen binding, "Second-generation" CARs include those that provide both co-stimulation (e.g., CD28 or CD137) and activation (CD3ζ). "Third-generation" CARs include those that provide multiple co-stimulation (e.g. CD28 and CD137) and activation (CD3ζ). A fourth generation of CARs have been described, CAR T cells redirected for cytokine killing (TRUCKS) where the vector containing the CAR construct possesses a cytokine cassette. When the CAR is ligated, the CAR T cell deposits a pro-inflammatory cytokine into the tumor lesion. A CAR-T cell is a T cell that expresses a chimeric antigen receptor. The phrase "chimeric antigen receptor (CAR)," as used herein and generally used in the art, refers to a recombinant fusion protein that has an antigen-specific extracellular domain coupled to an intracellular domain that directs the cell to perform a specialized function upon binding of an antigen to the extracellular domain. The terms "artificial T-cell receptor," "chimeric T-cell receptor," and "chimeric immunoreceptor" may each be used interchangeably herein with the term "chimeric antigen receptor."

As used herein, the term "cell" includes prokaryotic and eukaryotic cells. In one embodiment, a cell of the disclosure is a bacterial cell. In another embodiment, a cell of the disclosure is a fungal cell, such as a yeast cell. In another embodiment, a cell of the disclosure is a vertebrate cell, e.g., an avian or mammalian cell. In a preferred embodiment, a cell of the disclosure is a murine or human cell. As used herein, the term "engineered" (as in an engineered cell) refers to a cell into which a nucleic acid molecule e.g., encoding an IL-2 protein (e.g., a spliced and/or unspliced form of IL-2) or fragments thereof, has been introduced.

As used herein, the term "cell free composition" refers to an isolated composition, which does not contain intact cells. Examples of cell free compositions include cell extracts and compositions containing isolated proteins.

The term "combination therapy", as used herein, refers to those situations in which two or more different pharmaceutical agents are administered in overlapping regimens so that the subject is simultaneously exposed to both agents. When used in combination therapy, two or more different agents may be administered simultaneously or separately. This administration in combination can include simultaneous administration of the two or more agents in the same dosage form, simultaneous administration in separate dosage forms, and separate administration. That is, two or more agents can be formulated together in the same dosage form and administered simultaneously. Alternatively, two or more agents can be simultaneously administered, wherein the agents are present in separate formulations. In another alternative, a first agent can be administered just followed by one or more additional agents. In the separate administration protocol, two or more agents may be administered a few minutes apart, or a few hours apart, or a few days apart.

As used herein, the terms "comprising," "comprise" or "comprised," and variations thereof, in reference to defined or described elements of an item, composition, apparatus, method, process, system, etc. are meant to be inclusive or open ended, permitting additional elements, thereby indicating that the defined or described item, composition, apparatus, method, process, system, etc. includes those specified elements--or, as appropriate, equivalents thereof--and that other elements can be included and still fall within the scope/definition of the defined item, composition, apparatus, method, process, system, etc.

As used herein, the term "cytokine" refers generically to proteins released by one cell population that act on another cell as intercellular mediators or have an autocrine effect on the cells producing the proteins. Examples of such cytokines include lymphokines, monokines; interleukins ("ILs") such as IL-1, IL-1.alpha., IL-2, IL-3, IL-4, IL-5, IL-6, IL-7, IL-8, IL-9, IL-10, IL-11, IL-12, IL-13, IL-15, IL-17A-F, IL-18 to IL-29 (such as IL-23), IL-31, including PROLEUKIN^{™} rIL-2; a tumor-necrosis factor such as TNF-α or TNF-β, TGF-β1-3; and other polypeptide factors including leukemia inhibitory factor ("LIF"), ciliary neurotrophic factor ("CNTF"), CNTF-like cytokine ("CLC"), cardiotrophin ("CT"), and kit ligand ("KL").

"Diagnostic" or "diagnosed" means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

The term "expression vector" refers to a vector containing a nucleic acid sequence coding for at least part of a gene product capable of being transcribed. In some cases, when the transcription product is an mRNA molecule, this is in turn translated into a protein, polypeptide, or peptide.

As used herein, the term "immune checkpoint modulator" refers to an agent that interacts directly or indirectly with an immune checkpoint. In some embodiments, an immune checkpoint modulator increases an immune effector response (e.g., cytotoxic T cell response), for example by stimulating a positive signal for T cell activation. In some embodiments, an immune checkpoint modulator increases an immune effector response (e.g., cytotoxic T cell response), for example by inhibiting a negative signal for T cell activation (e.g. disinhibition). In some embodiments, an immune checkpoint modulator interferes with a signal for T cell anergy. In some embodiments, an immune checkpoint modulator reduces, removes, or prevents immune tolerance to one or more antigens.

"Interleukins" are a group of cytokines that play important roles in innate and adaptive immune system function. Some interleukins promote the development and differentiation of B lymphocytes, T lymphocytes, and hematopoietic cells. Many interleukins are produced by helper CD4 T lymphocytes, monocytes, macrophages, and endothelial cells. Interleukins can either enhance or inhibit immune function, depending on the particular interleukin.

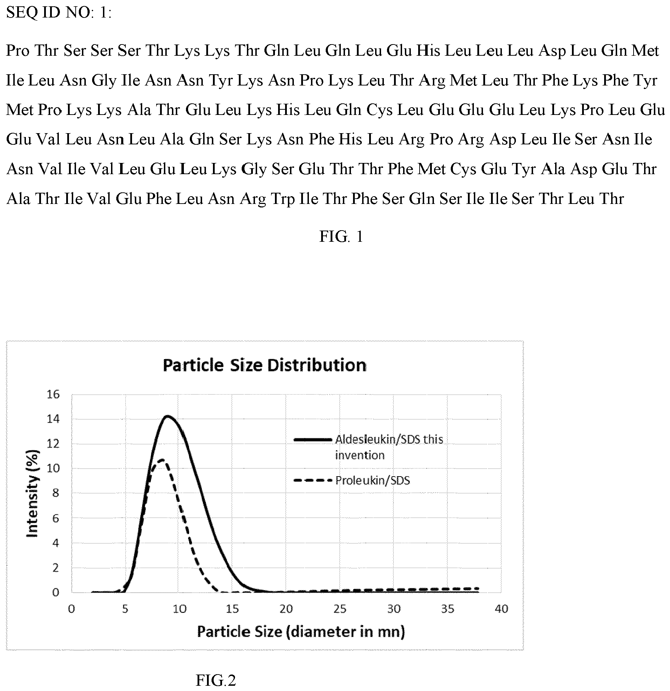

As used herein, the term human recombinant IL-2 (rhIL-2) is an unglycosylated protein produced by a microorganism that has been transfected with the human IL-2 codifying DNA sequence or a non-extensive modification of it. In fact, the IL-2 human recombinant version most used in clinical practices is modified version of the natural human IL-2 amino acid sequence, named aldesleukin (PROLEUKIN^{™}) having the following modifications: a) aldesleukin has no the N-terminal alanine present in natural IL-2 b) aldesleukin has serine substituted for cysteine at amino acid position 125. Aldesleukin amino acid sequence is shown in FIG. 1. Construction of the aldesleukin gene is described in US patent 4518584A and related non-US patents. The United States FDA has approved aldesleukin (PROLEUKIN^{™}) for use in the treatment of adults with metastatic renal cell carcinoma and metastatic melanoma. PROLEUKIN^{™} is supplied as a sterile, white to off-white, lyophilized cake in 20 single-use vials intended for intravenous administration. When reconstituted with 1.2 mL Sterile water for Injection, USP, each mL contains 18 million International Units (1.1 mg) PROLEUKIN^{™}, 50 mg mannitol, and 0.18 mg sodium dodecyl sulfate, buffered with approximately 0.17 mg monobasic and 0.89 mg dibasic sodium phosphate to a pH of 7.5 (range 7.2 to 7.8). Reconstituted or diluted PROLEUKIN^{™} is stable for up to 48 hours at refrigerated and room temperatures, 2° to 25°C (36° to 77°F) as stated in the Reconstitution and Dilution Directions of the "PROLEUKIN^{™} (aldesleukin) injection label - FDA" (Reference ID: 3165255).

As used herein, the term "interleukin 2" (IL-2) denotes a cytokine that regulates the activities of white blood cells (mainly lymphocytes). This cytokine is part of the immune response to microbial infections and exercise its effects by binding to specific receptors present on the surface of lymphocytes. IL-2 binds to IL-2 receptors (e.g., expressed by lymphocytes) and regulates the activity of white blood cells (e.g., leukocytes, lymphocytes), plays important roles in self versus non-self immune recognition, and participates in responses against microbial infectious agents. IL-2 promotes the differentiation of T cells into effector T cells and memory T cells, and also promotes the differentiation of immature T cells into regulatory T cells. In the context of cancer therapy, IL-2 can increase immune cell activation and subsequent immune cell-mediated inhibition of tumor cell growth.

Further information about IL-2 can be found in its "GeneCard" (accession number GC04M123831). Other accession numbers include: HGNC: 6001 Entrez Gene: 3558 Ensembl: ENSG00000109471 OMIM: 147680 UniProtKB: P60568. IL-2 proteins include native IL-2 proteins, as well as variant IL-2 proteins. A "native" or "wild type" IL-2 sequence, as used herein, refers to a human IL-2 sequence (e.g., Accession No. NP 000577.2), whether purified from natural sources or made using recombinant techniques. In some embodiments, an IL-2 sequence includes the PROLEUKIN^{™} (aldesleukin) sequence: (SEQ ID NO: 1):

PTSSSTKKTQ LQLEHLLLDL QMILNGINNY KNPKLTRMLT FKFYMPKKAT
ELKHLQCLEE ELKPLEEVLN LAQSKNFHLR PRDLISNINV IVLELKGSET
TFMCEYADET ATIVEFLNRW ITFSQSIIST LT

Human IL-2 sequence (e.g., Accession No. NP 000577.2) (SEQ ID NO: 2):

MYRMQLLSCI ALSLALVTNS APTSSSTKKT QLQLEHLLLD LQMILNGINN
YKNPKLTRML TFKFYMPKKA TELKHLQCLE EELKPLEEVL NLAQSKNFHL
RPRDLISNIN VIVLELKGSE TTFMCEYADE TATIVEFLNR WITFCQSIIS
TLT.

The term "immune response" refers to any response that is induced, triggered, or enhanced in a subject by an antigen (e.g., tumor cell antigen). The term includes the development, maturation, differentiation, and activation of immune cells (e.g., B cells and T cells), as well as the production of antibodies against an antigen. The term also includes increasing or decreasing the expression or activity of cytokines that are involved in regulating immune function.

As used herein, the term "in combination" in the context of the administration of a therapy to a subject refers to the use of more than one therapy for therapeutic benefit. The term "n combination" in the context of the administration can also refer to the prophylactic use of a therapy to a subject when used with at least one additional therapy. The use of the term "n combination" does not restrict the order in which the therapies (e.g., a first and second therapy) are administered to a subject. A therapy can be administered prior to (e.g., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy to a subject. The therapies are administered to a subject in a sequence and within a time interval such that the therapies can act together. In a particular embodiment, the therapies are administered to a subject in a sequence and within a time interval such that they provide an increased benefit than if they were administered otherwise. Any additional therapy can be administered in any order with the other additional therapy.

As used herein, the term "kit" refers to any delivery system for delivering materials. Inclusive of the term "kits" are kits for both research and clinical applications. In the context of reaction assays, such delivery systems include systems that allow for the storage, transport, or delivery of reaction reagents (e.g., cytokines, oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contains a subportion of the total kit components. The containers may be delivered to the intended recipient together or separately. For example, a first container may contain an enzyme for use in an assay, while a second container contains oligonucleotides or liposomes. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

The term "lymphocyte" refers to a subtype of white blood cells that includes T lymphocytes (also called "T cells"), B lymphocytes (also called "B cells"), dendritic cells, and natural killer (NK) cells. T lymphocytes mature in the thymus and play a central role in cell-mediated immunity. T lymphocytes are distinguished from other types of lymphocytes by the presence of T-cell receptors that are present on the cell surface. T lymphocytes are subdivided into several types, including effector T cells, helper T cells, cytotoxic (killer) T cells, memory cells, regulatory (suppressor) T cells, and natural killer T cells. Effector T cells refer to a broad category that includes several T lymphocyte types (including helper, killer, and regulatory cells), and generally are cells that respond to a stimulus. Helper T cells, also known as CD4⁺T cells, assist other types of white blood cells, playing roles such as assisting with the maturation of B cells into plasma and memory B cells, and activation of cytotoxic T cells and macrophages. Helper T cells are activated by the presentation of an antigen on the surface of an antigen-presenting cell (APC). Cytotoxic (killer) T cells, also known as CD8.sup.+ T cells, destroy virus-infected cells and tumor cells, and are associated with transplant rejection. Memory T cells recognize foreign invaders such as bacteria and viruses, and also tumor cells. After memory T cells encounter and respond to their cognate antigen, upon a second encounter, these cells are able to reproduce and mount a stronger and fast immune response. Regulatory (suppressor) T cells (Tregs) are critical for maintaining immunological tolerance; their major role is to downregulate T lymphocyte-mediated immunity near the end of an immune reaction and to inhibit autoreactive T lymphocytes that escaped the process of negative selection in the thymus. Natural killer T cells, which bridge the adaptive and innate immune systems, recognize an antigen presented by CD1d molecules and perform functions such as cytokine production and the release of cytolytic molecules. B lymphocytes secrete antibodies and function in the humoral immunity component of the adaptive immune system. In addition, B lymphocytes can function as antigen-presenting cells and secrete cytokines. B lymphocytes, which mature in the bone marrow, are distinguished from other types of lymphocytes by the presence of B cell receptors on the cell surface. Activation of B lymphocytes can either be T lymphocyte-dependent or T lymphocyte-independent. B lymphocytes are subdivided into several types, including plasmablast cells, plasma cells, lymphoplasmacytoid cells, memory B cells, follicular B cells, marginal zone B cells, B-1 cells, B-2 cells, and regulatory B (Breg) cells. NK cells, which are part of the innate immune system, play major roles in defending against tumor cells and virally infected cells, differentiating these cells from normal cells by recognizing cell surface changes in major histocompatibility complex class I molecules. NK cells are activated by the interferon family of cytokines, and once activated release cytotoxic molecules that destroy the altered or infected cells.

The term "modulator" is used to refer to an entity whose presence in a system in which an activity of interest is observed correlates with a change in level and/or nature of that activity as compared with that observed under otherwise comparable conditions when the modulator is absent. In some embodiments, a modulator is an activator, in that activity is increased in its presence as compared with that observed under otherwise comparable conditions when the modulator is absent. In some embodiments, a modulator is an inhibitor, in that activity is reduced in its presence as compared with otherwise comparable conditions when the modulator is absent. In some embodiments, a modulator interacts directly with a target entity whose activity is of interest. In some embodiments, a modulator interacts indirectly (i.e., directly with an intermediate agent that interacts with the target entity) with a target entity whose activity is of interest. In some embodiments, a modulator affects level of a target entity of interest; alternatively or additionally, in some embodiments, a modulator affects activity of a target entity of interest without affecting level of the target entity. In some embodiments, a modulator affects both level and activity of a target entity of interest, so that an observed difference in activity is not entirely explained by or commensurate with an observed difference in level.

As used in this specification and the appended claims, the term "or" is generally employed in its sense including "and/or" unless the content clearly dictates otherwise.

The term "peripheral blood mononuclear cell (PBMC)" refers to any peripheral blood having a round nucleus. PBMCs include lymphocytes (e.g., T lymphocytes, B lymphocytes, NK cells) and monocytes. PBMCs can be extracted from whole blood, for example, by using the polysaccharide Ficoll, followed by gradient centrifugation. Following centrifugation, PBMCs are found between the top layer of plasma and the bottom fraction of polymorphonuclear cells and erythrocytes.

The phrase "pharmaceutically acceptable carrier" refers to a carrier for the administration of a therapeutic agent. Exemplary carriers include saline, buffered saline, dextrose, water, glycerol, ethanol, and combinations thereof. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

As used herein, the term "secondary active agent", refers to any molecule that is used for the prevention or treatment of a virus infection, *e.g.* a respiratory virus infection, and include agents which alleviate any symptom, such as runny nose, blocked nose, sore throat, sneezing, chilliness, headache, muscle ache, cough, etc., associated with the virus. The term is inclusive of agents which alleviate any symptoms associated with the virus, for example, anti-pyretic agents, anti-inflammatory agents, chemotherapeutic agents, and the like. The "secondary active agent" can be a compound that is directly or indirectly effective in specifically interfering with at least one viral action, such as for example, virus penetration of eukaryotic cells, virus replication in eukaryotic cells, virus assembly, virus release from infected eukaryotic cells, or that is effective in inhibiting a virus titer increase or in reducing a virus titer level in a eukaryotic or mammalian host system. It also refers to a compound that prevents from or reduces the likelihood of getting a viral infection. The term includes, without limitation: antibodies, aptamers, adjuvants, anti-sense oligonucleotides, chemokines, cytokines, immune stimulating agents, immune modulating agents, B-cell modulators, T-cell modulators, NK cell modulators, antigen presenting cell modulators, enzymes, siRNA's, ribavirin, protease inhibitors, helicase inhibitors, polymerase inhibitors, helicase inhibitors, neuraminidase inhibitors, nucleoside reverse transcriptase inhibitors, non-nucleoside reverse transcriptase inhibitors, purine nucleosides, chemokine receptor antagonists, interleukins, nucleoside reverse transcriptase inhibitors (NRTIs), analogs, variants etc. or combinations thereof.

As used herein, "stable" or "highly stable" refers to the activity and/or the integrity of the molecule, e.g. IL-2, in the formulations embodied herein, over extended periods of time, as evaluated by biological activity *in vitro,* structural studies *in vitro* and therapeutic activity *in vivo.* The activity of the IL-2 can be measured by any standard assay. The activity before storage and after long-term storage, e.g. at least one year, can be compared. Highly stable IL-2 would have the same or slightly less activity as compared to the activity at the time of IL-2 when first formulated.

The terms "subject", "patient" or "individual" are used interchangeably herein, and refers to a mammalian subject to be treated, with human patients being preferred. In some cases, the methods of the invention find use in experimental animals, in veterinary application, and in the development of animal models for disease, including, but not limited to, rodents including mice, rats, and hamsters; and primates. Patients in need of therapy comprise those at risk of developing a certain condition, disease or disorder (e.g. due to genetic, environmental or physical attributes, such as for example, obesity). Patients in need of therapy also include those afflicted with a condition, disease or disorder. The diseases or disorders comprise, for example: autoimmune diseases, cancer, inflammatory diseases, neurological diseases or disorders, neuroinflammatory diseases or disorders, cardiovascular disease, obesity, diseases or disorders caused by infectious agents such as, for example, viruses, bacteria, fungi, prions, or parasites.

As used herein, the terms "treat," treating," "treatment," and the like refer to reducing or ameliorating a disorder and/or symptoms associated therewith. It will be appreciated that, although not precluded, treating a disorder or condition does not require that the disorder, condition or symptoms associated therewith be completely eliminated. "Treatment" of any disease mentioned herein encompasses an alleviation of at least one symptom of the disease, a reduction in the severity of the disease, or the delay or prevention of disease progression to more serious symptoms that may, in some cases, accompany the disease or to at least one other disease. Treatment need not mean that the disease is totally cured. A useful therapeutic agent needs only to reduce the severity of a disease, reduce the severity of symptom(s) associated with the disease or its treatment, or delay the onset of more serious symptoms or a more serious disease that can occur with some frequency following the treated condition. For example, if the disease is an inflammatory bowel disease, a therapeutic agent may reduce the number of distinct sites of inflammation in the gut, the total extent of the gut affected, reduce pain and/or swelling, reduce symptoms such as diarrhea, constipation, or vomiting, and/or prevent perforation of the gut. A patient's condition can be assessed by standard techniques such as an X-ray performed following a barium enema or enteroclysis, endoscopy, colonoscopy, and/or a biopsy. Suitable procedures vary according to the patient's condition and symptoms.

"Tumor," as used herein, refers to all neoplastic cell growth and proliferation, whether malignant or benign, and all pre-cancerous and cancerous cells and tissues. The terms "cancer", "cancerous", "cell proliferative disorder", "proliferative disorder" and "tumor" are not mutually exclusive as referred to herein.

Genbank and NCBI submissions indicated by accession number cited herein are incorporated herein by reference. All other published references, documents, manuscripts and scientific literature cited herein are incorporated herein by reference. In the case of conflict, the present specification, including definitions, will control. In addition, the materials, methods, and examples are illustrative only and not intended to be limiting.

Ranges: throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Methods of Producing Stable IL-2 Formulations

The disclosure is based in part on a novel method for preparing aldesleukin, which results in a product fulfilling all the structural and biological characteristics of PROLEUKIN^{™} but that surprisingly is very stable (at least one year) in solution as evaluated by biological activity "*in vitro*", structural studies "*in vitro*" and therapeutic activity "*in vivo*". An exemplary method to prepare aldesleukin largely stable in solution comprises the following steps:
a) Fermentation of an *E. coli* strain transfected with an expression vector engineered to highly express the aldesleukin gene. Bacteria are preferred microorganisms for producing IL-2 and among bacteria *E. coli* strains are the most preferred. *Escherichia coli* B serves as a research model and also for protein expression in life science laboratories and in the biotech industry. Characteristics such as protease deficiency, low acetate production at a high level of glucose, and enhanced permeability (probably due to a simple cell surface) make *E. coli* B a desirable host for the production of genetically engineered proteins. Differences between B strains and K12 include the absence of flagellar component genes, the DNA cytosine methylase dcm, and ompT in BL21(DE3). B strains may have an additional type II secretion system not found in K12. BL21(DE3) also carries a DE3 recombinant phage harboring the T7 RNA polymerase gene that can direct high-level expression of cloned genes under the control of the T7 promoter. Typical *E. coli* strains used for recombinant protein expression are: BL21 (a B *E. coli* strain that protects target protein from Ion and ompT proteases) and their derivatives such as: Lysogenic DE3 (based on T7 polymerase), pLysS, pLysE (express T7 lysozyme reducing basal expression of target genes), Origami (allows disulfide bond formation in *E. coli* cytoplasm), Rosetta (enhances expression of proteins that contain codons rarely used in *E. coli*). Similar versions exists under the K12 *E. coli* genetic background. Typical plasmid vectors for high expression of recombinant proteins in E.coli are: pET series based on pBR322 origin and T7/lac promoters; pBad with araBAD promoter and pUC origin; pGEX with tac promoter and pBR322 origin also. Combination of fusion tags sequences, protease cleavage sites, selection markers and strain compatibility are source for the most usual list of high expression plasmid variants.

Expression vectors are commercially available and a DNA sequence coding for the aldesleukin amino-acid sequence is inserted into the vector. The cells used in the instant assays can be eukaryotic or prokaryotic in origin. For example, in one embodiment, the cell is a bacterial cell. In another embodiment, the cell is a fungal cell, e.g., a yeast cell. In another embodiment, the cell is a vertebrate cell, e.g., an avian or a mammalian cell. In a another embodiment, the cell is a human cell. The cells of the disclosure can express endogenous IL-2 or fragments thereof, or can be engineered to do so. For example, a cell that has been engineered to express the IL-2 or fragments thereof can be produced by introducing into the cell an expression vector encoding the protein.

In certain embodiments, the cell is an *E. coli* cell. Different *E*. *coli* strains can be transfected in order to obtain optimal aldesleukin production.
a) Aldesleukin producing bacteria can be cultured in a suitable growth medium. For example, the medium may contain each 9 liters, 216 gr of Yeast Extract, 108 gr of Soy Peptone, 113 of gr K₂HPO₄, 20.8 gr KH₂PO₄, 36 ml of Glycerol and 4 ml of Antifoam (2% v/v). Fermentation conditions may be: Temperature: 37°C ± 0.5°C, agitation: 350 rpm ± 10 rpm, air flow: 9 L/min ± 1 L/min, pO2: set point 40%, agitation cascade: between 21% and 36% running between 350 rpm and 440 rpm, pH between 6.95 and 7.5 and inlet air pressure at 2 bar before starting Oxygen flow. After this, a feeding procedure should be followed. For example, feeding with a glucose solution 40% p/v by drip, to maintain a concentration of 0.1 %. Once the OD600 reaches 5 to 10, an appropriated inducer such as Isopropil-β-D-1-tiogalactopiranosido (IPTG) should be added to reach an operative concentration. At this point, the feeding with Glucose may be reduced to keep a glucose concentration of about 0.01%. Fermentation can be stop usually about 18 to 24 hours after inoculation. After fermentation, bacteria can be concentrated 5 to 7 times by centrifugation or tangential filtration and processed immediately or preserved at 2-8 °C (no more than 24 hours) or preserved at -20°C (for more than 24 hours).
b) After culture, aldesleukin is inside the bacteria, predominantly in the form aggregates named inclusion bodies (Ib). These Ib can be isolated by disruption of bacteria (for example by sonication). For this, bacteria could be suspended in purified water at temperature between 17 and 22°C before starting the disruption process. After this, the suspension could be circulated 2-3 times for the disruptor at a pressure of about 1400 bar. The lysate should be processed immediately or preserved at -20°C. The Ib are separated of other components of the lysate by centrifugation or tangential filtration and washed. The final buffer for the Ib washing may be TE (10 mM Tris-HCl, 1 mM disodium EDTA, pH 8.0). The Ib preparation should be stored at -20°C until further processing. The Ib are then suspended in a buffer containing 10 mM sodium Phosphate, 1% SDS, pH8.0 and stirred for 1hour. Then, an oxidation solution of cupric chloride is added to reach 100 µM final concentration and stirring should continue for 2 more hours. After this EDTA should be added to reach a 100 µM final concentration. The Mixture should be store at 2-8 °C until used in the downstream process.
c) The first chromatography is carried out using a ceramic hydroxyapatite (Type I 80 µm Bio-Rad) column. The aldesleukin preparation is loaded, washed with a solution 100 mM sodium phosphate, 0.3% SDS, pH 6.5 and eluded with a solution 250 mM sodium phosphate, 0.3%, pH 6.5.
d) The second chromatography is carried out using an HPLC C4 column. The solution containing the aldesleukin recovered from the first chromatography is mixed with acetonitrile (9 parts of the chromatography I pool elution and one part of Acetonitrile). After loaded aldesleukin is eluted using a gradient of Acetonitrile. Fractions containing aldesleukin are collected in a solution containing 73 mM sodium phosphate, 0.3 % SDS, pH 7.4.
e) The last step in aldesleukin purification is a diafiltration using 5kD cassettes. Aldesleukin is equilibrated using a pH 7 - 8 phosphate buffer. The buffer may contain up to 1 % SDS and up to 50 mg/mL mannitol, depending on the concentration of IL-2 of the solution obtained before this step.

Surprisingly it was found that the aldesleukin/SDS complexes obtained by the production method of the disclosure do not need to be lyophilized as described in EP 1 688 146 B1 to be stabilized, since they are stable in solution for at least one year as described in Examples 4 and 6.

It should be pointed out that the method disclosed herein, is different from the one taught by EP 1 688 146 B1. Some major differences are:
- After dissolution of inclusion bodies, no precipitation step is needed for the purification of IL-2 in the method disclosed herein, as is the case in EP 1 688 146 B1.
- Only two chromatographic steps are used in the process disclosed herein, whereas the process disclosed in EP 1 688 146 B1 includes three chromatographic steps.
- Hydroxyapatite is used herein, as the first chromatographic step, but this stationary phase is never used in EP 1 688 146 B1.
- Since the process herein does not need to precipitate IL-2, there is therefore no need to re-solubilize it using high levels of SDS. This difference is particularly important, because this step is considered critical for the formation of the IL-2/SDS microaggregates taught by EP 1 688 146 B1.
In conclusion, the manufacturing process of the disclosure provides a different way of obtaining IL-2 / SDS therapeutically active microaggregates with less chromatographic steps and without the need of precipitation and re-solubilization of IL-2 during downstream.

According to the dynamic light scattering assays shown in examples 3 and 4, the aldesleukin / SDS aggregates obtained by the production method of the disclosure have an extended size range between 4 and 18 nm with a peak at about 8 nm. Even though reconstituted PROLEUKIN^{™} also shows a similar distribution curve, the area under this curve is clearly lower than the area under the curve of the aldesleukin / SDS particles prepared by the method of the disclosure. Since the protein mass used in both cases is the same, this result suggests that upon reconstitution, part of the PROLEUKIN^{™} may remain as large aggregates. In fact, in order to measure the full activity of PROLEUKIN^{™}, it has been reported that the lyophilized preparation should be suspended in an SDS solution to avoid aggregate formation (6).

WO 2017068031 A1 describes a liquid pharmaceutical composition suitable for injection to a patient, consisting essentially of interleukin-2 at a concentration of 0.1 to 20 million IU/mL can be obtained. This composition contains an anionic surfactant in order to maintain stability in solution the interleukin 2 should, such as sodium dodecyl sulfate (SDS) that may be present at a concentration of about 0.05 to 0.5 mg/ml. Therefore, the microaggregation state and the therapeutic activity of any novel IL-2 composition should be assessed. Regarding this, Examples 3 and 4 show the microaggregation state and stability, respectively. On the other hand, Example 6 describes an assay to assess the therapeutic value of the aldesleukin/SDS aggregates obtained by the method of the disclosure. Example 6 is an illustration of the therapeutic activity of these microaggregates in medical treatments involving the use of cells expressing IL-2 receptors in their membranes. Examples of these cells already known in the art are Natural Killer (NK) and T cells.

### Therapeutics

It is well established that immunotherapy, including IL-2, is an effective approach for enhancing anti-tumor immunity against certain types of cancer. IL-2 has stimulatory effects on a number of immune cell types including T and B cells, monocytes, macrophages, lymphokine-activated killer cells (LAK) and NK cells (Waldmann, T. A., Nat Rev Immunol, 6: 595-601, 2006). Based on its ability to provide durable curative antitumor responses, systemic administration of recombinant human IL-2 (PROLEUKIN^{®}) has been approved to treat patients with metastatic melanoma or renal cell carcinoma (Rosenberg, S. A. et al., Ann Surg, 210: 474-484; discussion 484-475, 1989; Fyfe, G. et al., J Clin Oncol, 13: 688-696, 1995; and Atkins, M. B. et al., J Clin Oncol, 17: 2105-2116, 1999). Unfortunately, the considerable toxicity associated with this treatment makes it difficult to achieve an effective dose at the site of the tumor and limits the population that can be treated. For example, systemic treatment with IL-2 at tolerated doses induces lymphoid activation in virtually all treated patients, but anti-tumor responses are observed in a minority of these individuals (Rosenberg, S. A. et al., Ann Surg, 210: 474-484; discussion 484-475, 1989). As a result, use of high dose IL-2 is limited to specialized programs with experienced personnel and it is generally offered to patients who are responsive and have excellent organ function (Tarhini, A. A. et al., Curr Opin Investig Drugs, 6: 1234-1239, 2005). Local treatment (intravesical) of superficial bladder cancer patient with IL-2 has been shown to provide tumor regression and prolonged regression free time in a number of clinical studies (Den Otter, W. et al., J Urol, 159: 1183-1186, 1998; and Den Otter, W. et al., Cancer Immunol Immunother, 57: 931-950, 2008). In a Phase 2 study, systemic IL-2 administration to patients with cisplatin-refractory advance/metastatic urothelial carcinoma (65% of which were bladder cancers) provided a median survival of over 10 months compared to 6-7 months observed using single agent or combination salvage chemotherapy, suggesting further evidence of bladder carcinoma sensitivity to IL-2 therapy (Kim, J. et al., Urol Oncol, 21: 21-26, 2003; and Gallagher, D. J. et al., Cancer, 113: 1284-1293, 2008). However, IL-2 induced toxicities in these patients were significant and limited the treatment regimen (Kim, J. et al., Urol Oncol, 21: 21-26, 2003). Thus, there is a critical need for innovative strategies that enhance the curative effects of IL-2, reduce its toxicity without compromising clinical benefit and expand its utility beyond the currently approved indications.

Accordingly, the therapeutic methods of the invention (which include prophylactic treatment) in general comprise administration of a therapeutically effective amount of IL-2, *e.g.* SEQ ID NO: 1, to a subject (e.g., animal, human) in need thereof, including a mammal, particularly a human. Determination of those subjects "at risk" can be made by any objective or subjective determination by a diagnostic test or opinion of a subject or health care provider (e.g., genetic test, enzyme or protein marker, Marker (as defined herein), family history, and the like).

In certain embodiments, cells from patients are obtained and cultured with the IL-2 compositions embodied herein, expanded and re-infused to the patient.

Regulatory T cells (Tregs) are important in the maintenance of immune cell homeostasis as evidenced by the catastrophic consequences of genetic or physical ablation of the Treg population. Specifically, Treg cells maintain order in the immune system by enforcing a dominant negative regulation on other immune cells. Broadly classified into natural or adaptive (induced) Tregs; natural Tregs are CD4⁺CD25⁺ T-cells which develop and emigrate from the thymus to perform their key role in immune homeostasis. Adaptive Tregs are non-regulatory CD4⁺ T-cells which acquire CD25 (IL-2R alpha) expression outside of the thymus, and are typically induced by inflammation and disease processes, such as autoimmunity and cancer.

There is increasing evidence that Tregs manifest their function through a myriad of mechanisms that include the secretion of immunosuppressive soluble factors such as IL-9, IL-10 and TGF beta, cell-contact-mediated regulation via the high affinity TCR and other costimulatory molecules such as CTLA-4, GITR, and cytolytic activity. Under the influence of TGF beta, adaptive Treg cells mature in peripheral sites, including mucosa-associated lymphoid tissue (MALT), from CD4⁺ Treg precursors, where they acquire the expression of markers typical of Tregs, including CD25, CTLA4 and GITR / AITR. Upon up-regulation of the transcription factor Foxp3, Treg cells begin their suppressive effect. This includes the secretion of cytokines including IL-10 and TGF beta which may induce cell-cycle arrest or apoptosis in effector T cells, and blocking co-stimulation and maturation of dendritic cells.

In some embodiments, the pharmaceutical compositions increase the regulatory T cell (Treg) count (i.e. proliferation) when administered to a subject. For example, the pharmaceutical compositions comprising the IL-2 embodied herein (*e.g.* SEQ ID NO: 1) of the present disclosure can increase the regulatory T cell count in a subject after administration to the subject relative to the subject's regulatory T cell count before administration. For example, administration of a pharmaceutical composition comprising the IL-2 molecules of the present disclosure to a subject may increase the levels of regulatory T cells in the subject by at least 1-fold, 2-fold, 3-fold, 4-fold, 5-fold, 6-fold,7-fold, 8-fold, 9-fold, 10-fold, 15-fold, 20-fold, 25-fold, 30-fold,35-fold, 40-fold, 45-fold, 50-fold, 55-fold or 60-fold relative to the subject's regulatory T cell count before administration.

In some embodiments, the pharmaceutical composition comprising the IL-2 molecules of the present disclosure can increase the regulatory T cell count in a subject after administration to the subject relative to administration of the IL-2 (e.g. aldesleukin; SEQ ID NO: 1). Aldesleukin is an IL-2 variant comprising a C125S substitution relative to human wildtype IL-2 (SEQ ID NO: 2) in which the N-terminal alanine has been removed. Administration of a pharmaceutical composition comprising the molecules of the present disclosure to a subject may increase the levels of regulatory T cells in the subject by at least 10%, 20%, 30%, 40%, 50%, 100%, 150%, 200%, 250%, 300%, 350%,400%, 450% or 500% relative to an equimolar amount of IL-2 (e.g. aldesleukin).

In some embodiments, the molecules of the present disclosure can increase the ratio of regulatory T cells (Treg) to conventional T cells (Tconv) in a subject. In humans, daily low dose IL-2 therapy has been used to treat patients with chronic GVHD by augmenting the levels of Tregs (See Koreth J, et al., Blood. 2016 Jul. 7; 128(1):130-7; and Koreth J. N Engl J Med. 2011 Dec. 1; 365(22):2055-66). In the latter trial, IL-2 (aldesleukin) was administered by daily subcutaneous injection for 12 weeks. Overall, patients in these trials attained a greater than 5 fold increase of Tregs over baseline (Treg levels prior to treatment), a greater than 5 fold increase in their Treg/Tconv ratios, and a 61% clinical response rate. Clinical responses were strongly associated with a Treg/Tconv ratio ≥.0.2 at the end of the first week of treatment, which was an approximately 2.9 fold increase over the baseline Treg/Tconv ratios. Accordingly, in some embodiments, administration of a pharmaceutical composition comprising the IL-2 molecules of the present disclosure to a subject may increase the Treg/Tconv ratio to at least 0.2, 0.3, 0.4, 0.5, 0.6. 0.7, 0.8, 0.9, 1.0, 1.1, 1.2, 1.3,1.4 or 1.5. The Treg/Tconv ratio may be maintained at the levels described above over several doses of the IL-2 molecules of the present disclosure, for example over at least 2, 3, 4, 5, 6, 7, 8, 9, 10, 11, 12,13, 14, 15, 16, 17, 18, 19 or 20 doses.

The IL-2 molecules of the present disclosure may also increase regulatory T cell activity (e.g. immunosuppressive activity). Regulatory T cell function may be determined by measuring the expression of one or more biomarkers, including but not limited to, CD25, FOXP3, CTLA-4, ICOS and CD39. The level of FOXP3, a protein that is necessary for Treg function, is correlated with highly suppressive Tregs (Miyara M, et al., Immunity. 2009 Jun. 19; 30(6):899-911). The level of CD25 (IL2RA protein) has been associated with increased IL-2 consumption, a major immunosuppressive mechanism of Tregs (Chinen T, et al., Nat Immunol. 2016 November; 17(11):1322-1333).

*Adoptive cell therapy.* Adoptive cell therapy (ACT) (including allogeneic and autologous hematopoietic stem cell transplantation (HSCT) and recombinant cell (i.e., CAR-T) therapies) is the treatment of choice for many malignant disorders (for reviews of HSCT and adoptive cell therapy approaches, see, Rager & Porter, Ther Adv Hematol (2011) 2(6) 409-428; Roddie & Peggs, Expert Opin. Biol. Ther. (2011) 11(4):473-487; Wang et al. Int. J. Cancer: (2015)136, 1751-1768; and Chang, Y.J. and X.J. Huang, Blood Rev, 2013. 27(1): 55-62). Such adoptive cell therapies include, but are not limited to, allogeneic and autologous hematopoietic stem cell transplantation, donor leukocyte (or lymphocyte) infusion (DLI), adoptive transfer of tumor infiltrating lymphocytes, or adoptive transfer of T cells or NK cells (including recombinant cells, i.e., CAR-T, CAR-NK). Beyond the necessity for donor-derived cells to reconstitute hematopoiesis after radiation and chemotherapy, immunologic reconstitution from transferred cells is important for the elimination of residual tumor cells. The efficacy of ACT as a curative option for malignancies is influenced by a number of factors including the origin, composition and phenotype (lymphocyte subset, activation status) of the donor cells, the underlying disease, the pre-transplant conditioning regimen and post-transplant immune support (i.e., IL-2 therapy) and the graft-versus-tumor (GVT) effect mediated by donor cells within the graft. Additionally, these factors must be balanced against transplant-related mortality, typically arising from the conditioning regimen and/or excessive immune activity of donor cells within the host (i.e., graft-versus-host disease, cytokine release syndrome, etc.).

The cells, *e.g.* CAR-T cells, NK cells, T cells etc., once they have been expanded ex *vivo* in response to, for example, a tumor or viral antigen, are reinfused into the subject in a therapeutically effective amount. The term "therapeutically effective amount" as used herein means the amount of ells when administered to a mammal, in particular a human, in need of such treatment, is sufficient to treat autoimmune diseases such as rheumatoid arthritis, cancer and the like.

The precise amount of cells to be administered can be determined by a physician with consideration of individual differences in age, weight, extent of disease and condition of the subj ect.

Typically, administration of T cell therapies is defined by number of cells per kilogram of body weight. However, because T cells will replicate and expand after transfer, the administered cell dose will not resemble the final steady-state number of cells.

In an embodiment, a pharmaceutical composition comprising expanded cells, *e.g.* CAR-T cells may be administered at a dosage of 10⁴ to 10⁹ cells/kg body weight. In another embodiment, a pharmaceutical composition comprising the cells may be administered at a dosage of 10⁵ to 10⁶ cells/kg body weight, including all integer values within those ranges.

Compositions comprising the cells may also be administered multiple times at these dosages. The cells can be administered by using infusion techniques that are known in the art (see, for example, Rosenberg et al., 1988, New England Journal of Medicine, 319: 1676). The optimal dosage and treatment regimen for a particular subject can be readily determined by one skilled in the art by monitoring the patient for signs of disease and adjusting the treatment accordingly.

In certain embodiments, administration of any of the compositions embodied herein, e.g. a chimeric antigen receptor (CAR)-T cell, comprising a single chain variable fragment (scFv) which specifically binds to an immune checkpoint protein or receptors thereof, and a chimeric antigen receptor comprising an antigen binding domain; a hinge region; a transmembrane domain; a costimulatory domain; a cytoplasmic signaling domain. T cells for the treatment of cancer or virus infection, can be combined with other cell-based therapies, for example, stem cells, antigen presenting cells, etc.

Approaches utilizing adoptive NK cell therapy have become of significant interest. In patients receiving autologous HSCT, blood NK cell numbers recover very early after the transplant and the levels of NK cells correlate with a positive outcome (Rueff et al., 2014, Biol. BloodMarrow Transplant. 20, 896-899). Although therapeutic strategies with autologous NK cell transfer have had limited success due to a number of factors, adoptive transfer *of ex vivo* activated allogeneic (or haplo-identical) NK cells has emerged as a promising immunotherapeutic strategy for cancer (Guillerey et al. 2016. Nature Immunol. 17: 1025-1036). The activity of these cells is less likely to be suppressed by self-MHC molecules compared to autologous NK cells. A number of studies have shown that adoptive therapy with haploidentical NK cells to exploit alloreactivity against tumor cells is safe and can mediate significant clinical activity in AML patients. Taking these findings further, recent studies have focused on optimizing *ex vivo* activation/expansion methods for NK cells or NK precursors (i.e., stem cells) and pre-transplant conditioning and post-transplant immune support strategies; use of NK cell lines or recombinant tumor-targeting NK cells; evaluation of combination therapies with other agents such as therapeutic antibodies, immunomodulatory agents (lenalidomide), and anti-KIR and checkpoint antibodies.

Accordingly, in certain embodiments an NK cell is transfected with a chimeric antigen receptor. In certain embodiments, a CAR-NK cell comprises an antigen binding molecule e.g. an scFv, which specifically binds to an immune checkpoint protein or receptors thereof, and a chimeric antigen receptor comprising an antigen binding domain; a hinge region; a transmembrane domain; a costimulatory domain; a cytoplasmic signaling domain. As indicated herein, *ex vivo* incubation of CAR-NK cells can be activated, e.g. an IL-15 superagonist.

NK cells mediate some of their functions through the secretion of cytokines, such as interferon γ (IFN-γ), granulocyte-macrophage colony-stimulating factors (GM-CSFs), tumor necrosis factor α (TNF-α), macrophage colony-stimulating factor (M-CSF), interleukin-3 (IL-3), and IL-8. NK cell cytotoxic activity is regulated through a balance of activating and inhibitory receptors that enables fine-tuned control of cytotoxic activity, preventing cytotoxicity against healthy cells, while maintaining effective cytotoxic capacity against tumor cells. Indeed, multiple studies have demonstrated the safety of adoptive NK cell transfer and clinical anti-cancer effects, highlighting the potential for NK cells as an effective cancer immunotherapy ((Parkhurst, M. R., et al. Clin Cancer Res 17, 6287-6297 (2011); Ruggeri, L. et al. Science 295, 2097-2100, (2002); Miller, J. S. et al. Blood 105, 3051-3057, (2005; Bachanova, V. et al. Blood 123, 3855-3863, (2014); Rubnitz, J. E. et al. J Clin Oncol 28, 955-959, (2010)). For example, cytokines including IL-2, IL-12, TNF-α, and IL-1 can induce NK cells to produce cytokines. IFN-α and IL-2 are strong inducers of NK cell cytotoxic activity (G. Trinichieri et al., Journal of Experimental Medicine 160:1147-1169, 1984; G. Trinichieri and D. Santoli, Journal ofExperimental Medicine 147: 1314-1333, 1977). The presence of IL-2 both stimulates and expands NK cells (K. Oshimi, International Journal of Hematology 63:279-290, 1996). IL-12 has been shown to induce cytokine production from T and NK cells, and augment NK cell-mediated cytotoxicity (M. Kobayashi et al., Journal of Experimental Medicine 170:827-846, 1989).

NK cells have been expanded from multiple sources, including peripheral blood and umbilical cord blood (CB) ((Denman, C. J. et al. Membrane-bound IL-21 promotes sustained ex vivo proliferation of human natural killer cells. PLoS One 7, e30264, (2012); Knorr, D. A. et al. Clinical-scale derivation of natural killer cells from human pluripotent stem cells for cancer therapy. Stem Cells Transl Med 2, 274-283, (2013); Shah, N. et al. Antigen presenting cell-mediated expansion of human umbilical cord blood yields log-scale expansion of natural killer cells with anti-myeloma activity. PLoS One 8, e76781, (2013); Woll, P. S. et al. Human embryonic stem cells differentiate into a homogeneous population of natural killer cells with potent in vivo antitumor activity. Blood 113, 6094-6101, (2009)). *Ex vivo* NK cell expansion methods have been developed using cytokines in combination with artificial antigen-presenting cells (aAPCs) as feeder cells ((Denman, C. J. et al. PLoS One 7, e30264, (2012); Berg, M. et al. Cytotherapy 11, 341-355, (2009); Gong, W. et al. Tissue Antigens 76, 467-475, (2010); Zhang, H. et al., J Immunother 34, 187-195, (2011)).

In adoptive immunotherapy, the patient's circulating lymphocytes, or tumor infiltrated lymphocytes, are isolated in vitro, activated by lymphokines such as IL-2 or transduced with genes for tumor necrosis, and readministered. To achieve this, one would administer to an animal, or human patient, an immunologically effective amount of activated lymphocytes in combination with an adjuvant-incorporated antigenic peptide composition as described herein. The activated lymphocytes will most preferably be the patient's own cells that were earlier isolated from a blood or tumor sample and activated (or "expanded") *in vitro.* This form of immunotherapy has produced several cases of regression of melanoma and renal carcinoma, but the percentage of responders were few compared to those who did not respond. More recently, higher response rates have been observed when such adoptive immune cellular therapies have incorporated genetically engineered T cells that express chimeric antigen receptors (CAR) termed CAR T cell therapy. Similarly, natural killer cells both autologous and allogenic have been isolated, expanded and genetically modified to express receptors or ligands to facilitate their binding and killing of tumor cells.

The compositions of the present invention may be prepared in a manner known in the art and are those suitable for parenteral administration to mammals, particularly humans, comprising a therapeutically effective amount of the composition alone, with one or more pharmaceutically acceptable carriers or diluents.

The term "pharmaceutically acceptable carrier" as used herein means any suitable carriers, diluents or excipients. These include all aqueous and non-aqueous isotonic sterile injection solutions which may contain anti-oxidants, buffers and solutes, which render the composition isotonic with the blood of the intended recipient; aqueous and non-aqueous sterile suspensions, which may include suspending agents and thickening agents, dispersion media, antifungal and antibacterial agents, isotonic and absorption agents and the like. It will be understood that compositions of the invention may also include other supplementary physiologically active agents.

The carrier must be pharmaceutically "acceptable" in the sense of being compatible with the other ingredients of the composition and not injurious to the subject. Compositions include those suitable for parenteral administration, including subcutaneous, intramuscular, intravenous and intradermal administration. The compositions may conveniently be presented in unit dosage form and may be prepared by any method well known in the art of pharmacy. Such methods include preparing the carrier for association with the cells. In general, the compositions are prepared by uniformly and intimately bringing into association any active ingredients with liquid carriers.

In an embodiment, the composition is suitable for parenteral administration. In another embodiment, the composition is suitable for intravenous administration. Compositions suitable for parenteral administration include aqueous and nonaqueous isotonic sterile injection solutions which may contain anti-oxidants, buffers, bactericides and solutes, which render the composition isotonic with the blood of the intended recipient; and aqueous and non-aqueous sterile suspensions which may include suspending agents and thickening agents.

### Treatment of Autoimmune Diseases

The methods of this disclosure can be applied to various autoimmune or immune-related diseases or conditions, for example to treat such diseases or conditions. Autoimmune diseases include diseases that affect organs such as the heart, kidney, liver, lung, reproductive organs, digestive system, or skin. Autoimmune diseases include diseases that affect glands, including the endocrine, adrenal, thyroid, salivary and exocrine glands, and the pancreas. Autoimmune diseases can also be multi-glandular. Autoimmune diseases can target one or more tissues, for example connective tissue, muscle, or blood. Autoimmune diseases can target the nervous system or eyes, ears or vascular system. Autoimmune diseases can also be systemic, affecting multiple organs, tissues and/or systems. In some embodiments, an autoimmune disease or condition is an inflammatory disease or condition.

Non-limiting examples of autoimmune or immune-related diseases or conditions include inflammation, antiphospholipid syndrome, systemic lupus erythematosus, rheumatoid arthritis, autoimmune vasculitis, celiac disease, autoimmune thyroiditis, post-transfusion immunization, maternal-fetal incompatibility, transfusion reactions, immunological deficiency such IgA deficiency, common variable immunodeficiency, drug-induced lupus, diabetes mellitus, Type I diabetes, Type II diabetes, juvenile onset diabetes, juvenile rheumatoid arthritis, psoriatic arthritis, multiple sclerosis, immunodeficiency, allergies, asthma, psoriasis, atopic dermatitis, allergic contact dermatitis, chronic skin diseases, amyotrophic lateral sclerosis, chemotherapy-induced injury, graft-vs-host diseases, bone marrow transplant rejection, Ankylosing spondylitis, atopic eczema, Pemphigus, Behcet's disease, chronic fatigue syndrome fibromyalgia, chemotherapy-induced injury, myasthenia gravis, glomerulonephritis, allergic retinitis, systemic sclerosis, subacute cutaneous lupus erythematosus, cutaneous lupus erythematosus including chilblain lupus erythematosus, Sjogren's syndrome, autoimmune nephritis, autoimmune vasculitis, autoimmune hepatitis, autoimmune carditis, autoimmune encephalitis, autoimmune mediated hematological diseases, lc-SSc (limited cutaneous form of scleroderma), dc-SSc (diffused cutaneous form of scleroderma), autoimmune thyroiditis (AT), Grave's disease (GD), myasthenia gravis, multiple sclerosis (MS), ankylosing spondylitis, transplant rejection, immune aging, rheumatic/autoimmune diseases, mixed connective tissue disease, spondyloarthropathy, psoriasis, psoriatic arthritis, myositis, scleroderma, dermatomyositis, autoimmune vasculitis, mixed connective tissue disease, idiopathic thrombocytopenic purpura, Crohn's disease, human adjuvant disease, osteoarthritis, juvenile chronic arthritis, a spondyloarthropathy, an idiopathic inflammatory myopathy, systemic vasculitis, sarcoidosis, autoimmune hemolytic anemia, autoimmune thrombocytopenia, thyroiditis, immune-mediated renal disease, a demyelinating disease of the central or peripheral nervous system, idiopathic demyelinating polyneuropathy, Guillain-Barre syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease, psoriatic arthritis, psoriasis, dermatitis, polymyositis/dermatomyositis, toxic epidermal necrolysis, systemic scleroderma and sclerosis, responses associated with inflammatory bowel disease, ulcerative colitis, respiratory distress syndrome, adult respiratory distress syndrome (ARDS), meningitis, encephalitis, uveitis, colitis, glomerulonephritis, allergic conditions, eczema, conditions involving infiltration of T cells and chronic inflammatory responses, atherosclerosis, autoimmune myocarditis, leukocyte adhesion deficiency, allergic encephalomyelitis, immune responses associated with acute and delayed hypersensitivity mediated by cytokines and T-lymphocytes, tuberculosis, sarcoidosis, granulomatosis including Wegener's granulomatosis, agranulocytosis, vasculitis (including ANCA), aplastic anemia, Diamond Blackfan anemia, immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, central nervous system (CNS) inflammatory disorders, multiple organ injury syndrome, mysathenia gravis, antigen-antibody complex mediated diseases, anti-glomerular basement membrane disease, anti-phospholipid antibody syndrome, allergic neuritis, Bechet disease, Castleman's syndrome, Goodpasture's syndrome, Lambert-Eaton Myasthenic Syndrome, Reynaud's syndrome, Stevens-Johnson syndrome, pemphigoid bullous, pemphigus, autoimmune polyendocrinopathies, Reiter's disease, stiff-man syndrome, giant cell arteritis, immune complex nephritis, IgA nephropathy, IgM polyneuropathies or IgM mediated neuropathy, idiopathic thrombocytopenic purpura (ITP), thrombotic throbocytopenic purpura (TTP), autoimmune thrombocytopenia, autoimmune disease of the testis and ovary including autoimmune orchitis and oophoritis, primary hypothyroidism, autoimmune endocrine diseases including autoimmune thyroiditis, chronic thyroiditis (Hashimoto's Thyroiditis), subacute thyroiditis, idiopathic hypothyroidism, Addison's disease, autoimmune polyglandular syndromes (or polyglandular endocrinopathy syndromes), Sheehan's syndrome, autoimmune hepatitis, lymphoid interstitial pneumonitis (HIV), bronchiolitis obliterans (non-transplant) vs NSIP, large vessel vasculitis (including polymyalgia rheumatica and giant cell (Takayasu's) arteritis), medium vessel vasculitis (including Kawasaki's disease and polyarteritis nodosa), ankylosing spondylitis, Berger's disease (IgA nephropathy), rapidly progressive glomerulonephritis, primary biliary cirrhosis, Celiac sprue (gluten enteropathy), cryoglobulinemia, and amyotrophic lateral sclerosis (ALS).

### Methods of Treatment of Cancer

In some embodiments, described herein is a method of treating a proliferative disease or condition in a subject in need thereof, which comprises administering to the subject a therapeutically effective amount of the pharmaceutical compositions comprising the IL-2 molecule, *e.g.* SEQ ID NO: 1.

In certain embodiments, the IL-2 molecule described herein, *e.g.* SEQ ID NO: 1, interacts with the IL-2 receptor to stimulate and/or enhance expansion of CD4⁺ helper cells, CD8⁺ effector naive and memory T cells, NK cells, and/or NKT cells. In additional cases, the expansion of Teff cells skews the Teff:Treg ratio toward the Teff population which target tumor cells or cells infected with a pathogen, *e.g.* a virus.

In some embodiments, the proliferative disease or condition is a cancer. In some cases, the cancer is a solid tumor. Exemplary solid tumors include, but are not limited to, bladder cancer, bone cancer, brain cancer, breast cancer, colorectal cancer, esophageal cancer, eye cancer, head and neck cancer, kidney cancer, lung cancer, melanoma, ovarian cancer, pancreatic cancer, or prostate cancer. In some cases, the solid tumor is a metastatic cancer. In some cases, the solid tumor is a relapsed or refractory cancer. In some cases, the solid tumor is castrate-resistant prostate cancer, metastatic castrate-resistant prostate cancer, or metastatic castrate-resistant prostate cancer having DNA damage response (DDR) defects.

In some embodiments, the pharmaceutical composition comprising the IL-2 molecule embodied herein, is administered to a subject for the treatment of a metastatic cancer. In some instances, the metastatic cancer comprises a metastatic bladder cancer, metastatic bone cancer, metastatic brain cancer, metastatic breast cancer, metastatic colorectal cancer, metastatic esophageal cancer, metastatic eye cancer, metastatic head and neck cancer, metastatic kidney cancer, metastatic lung cancer, metastatic melanoma, metastatic ovarian cancer, metastatic pancreatic cancer, or metastatic prostate cancer. In some cases, the pharmaceutical composition comprising the IL-2 molecule embodied herein, is administered to a subject for the treatment of metastatic bladder cancer, metastatic bone cancer, metastatic brain cancer, metastatic breast cancer, metastatic colorectal cancer, metastatic esophageal cancer, metastatic eye cancer, metastatic head and neck cancer, metastatic kidney cancer, metastatic lung cancer, metastatic melanoma, metastatic ovarian cancer, metastatic pancreatic cancer, or metastatic prostate cancer. In some cases, the pharmaceutical composition comprising the IL-2 molecule embodied herein, is administered to a subject for the treatment of castrate-resistant prostate cancer, metastatic castrate-resistant prostate cancer, or metastatic castrate-resistant prostate cancer having DNA damage response (DDR) defects.

In some instances, the pharmaceutical composition comprising the IL-2 molecule embodied herein, is administered to a subject for the treatment of a relapsed or refractory cancer. In some instances, the relapsed or refractory cancer comprises a relapsed or refractory bladder cancer, relapsed or refractory bone cancer, relapsed or refractory brain cancer, relapsed or refractory breast cancer, relapsed or refractory colorectal cancer, relapsed or refractory esophageal cancer, relapsed or refractory eye cancer, relapsed or refractory head and neck cancer, relapsed or refractory kidney cancer, relapsed or refractory lung cancer, relapsed or refractory melanoma, relapsed or refractory ovarian cancer, relapsed or refractory pancreatic cancer, or relapsed or refractory prostate cancer. In some cases, the pharmaceutical composition comprising the IL-2 molecule embodied herein, is administered to a subject for the treatment of a relapsed or refractory bladder cancer, relapsed or refractory bone cancer, relapsed or refractory brain cancer, relapsed or refractory breast cancer, relapsed or refractory colorectal cancer, relapsed or refractory esophageal cancer, relapsed or refractory eye cancer, relapsed or refractory head and neck cancer, relapsed or refractory kidney cancer, relapsed or refractory lung cancer, relapsed or refractory melanoma, relapsed or refractory ovarian cancer, relapsed or refractory pancreatic cancer, or relapsed or refractory prostate cancer.

In some embodiments, the cancer is a treatment-naive cancer. In such cases, the treatment-naive cancer is a cancer that has not been treated by a therapy. In some cases, the treatment-naive cancer is a solid tumor, such as bladder cancer, a bone cancer, a brain cancer, a breast cancer, a colorectal cancer, an esophageal cancer, an eye cancer, a head and neck cancer, a kidney cancer, a lung cancer, a melanoma, an ovarian cancer, a pancreatic cancer, or a prostate cancer. In some embodiments, described herein is a method of treating a treatment-naive solid tumor in a subject in need thereof which comprises administering to the subject the pharmaceutical composition comprising the IL-2 molecule embodied herein.

In some embodiments, the cancer is a hematologic malignancy. In some instances, the hematologic malignancy comprises a leukemia, a lymphoma, or a myeloma. In some cases, the hematologic malignancy is a T-cell malignancy. In other cases, the hematological malignancy is a B-cell malignancy. Exemplary hematologic malignancies include, but are not limited to, chronic lymphocytic leukemia (CLL), small lymphocytic lymphoma (SLL), follicular lymphoma (FL), diffuse large B-cell lymphoma (DLBCL), mantle cell lymphoma (MCL), Waldenstrom's macroglobulinemia, multiple myeloma, extranodal marginal zone B cell lymphoma, nodal marginal zone B cell lymphoma, Burkitt's lymphoma, non-Burkitt high grade B cell lymphoma, primary mediastinal B-cell lymphoma (PMBL), immunoblastic large cell lymphoma, precursor B-lymphoblastic lymphoma, B cell prolymphocytic leukemia, lymphoplasmacytic lymphoma, splenic marginal zone lymphoma, plasma cell myeloma, plasmacytoma, mediastinal (thymic) large B cell lymphoma, intravascular large B cell lymphoma, primary effusion lymphoma, or lymphomatoid granulomatosis.

### Gene Therapy

More than 80 primary immune deficiency diseases are recognized by the World Health Organization. These diseases are characterized by an intrinsic defect in the immune system in which, in some cases, the body is unable to produce any enough, or effective antibodies against infection. In other cases, cellular defenses to fight infection fail to work properly. Typically, primary immune deficiencies are inherited disorders. Patients with inherited immune deficiencies such as adenosine deaminase deficient (ADA)-severe combined immunodeficiency (SCID), X-linked SCID, chronic granulomatous disease (CGD), Wiskott-Aldrich Syndrome, and Fanconi anemia (FA) can benefit from gene therapy, which provides a functioning gene to an affected patient to compensate for the defective one.

Secondary, or acquired, immune deficiencies are not the result of inherited genetic abnormalities, but rather occur in individuals in which the immune system is compromised by factors outside the immune system. Examples include trauma, viruses, chemotherapy, toxins, and pollution. Acquired immunodeficiency syndrome (AIDS) is an example of a secondary immune deficiency disorder caused by a virus, the human immunodeficiency virus (HIV), in which a depletion of T lymphocytes renders the body unable to fight infection. Patients with secondary immune deficiencies can also benefit from genetic therapies.

The compositions can be used in combination with one or more gene therapies related to primary immune deficiency diseases (PIDDS). These include, for example, Autoimmune Lymphoproliferative Syndrome (ALPS), APS-1 (APECED), CARD9, Chronic Granulomatous Disease (CGD), Congenital Neutropenia Syndromes, Common Variable Immunodeficiency (CVID), CTLA4 Deficiency, DOCK8 Deficiency, Glycosylation Disorders with Immunodeficiency, Hyper-Immunoglobulin E Syndromes (HIES), PI3 Kinase Disease, PLAID, Severe Combined Immunodeficiency (SCID), STAT3 Dominant-Negative Disease, WHIM Syndrome, X-Linked Agammaglobulinemia (XLA), X-Linked Lymphoproliferative Disease (XLP), Wiscott-Aldrich syndrome, DiGeorge syndrome, Ataxia-telangectasia, Chronic granulomatous disease, Transient hypogammaglobulinemia of infancy, Agammaglobulinemia, Complement deficiencies, Selective IgA deficiency.

X-linked severe combined immunodeficiency (SCID-X1) is both a cellular and humoral immune depletion caused by mutations in the common gamma chain gene (γC), which result in the absence of T and natural killer (NK) lymphocytes and the presence of nonfunctional B lymphocytes. SCID-X1 is fatal in the first two years of life unless the immune system is reconstituted, for example, through bone marrow transplant (BMT) or gene therapy. Administration of a therapeutic gene, for example, by an expression vector encoding a therapeutic protein, can be combined with a vector encoding IL-2, *e.g.* SEQ ID NOS: 1 and/or 2, or a pharmaceutical composition embodied herein. Particular examples of therapeutic genes and/or gene products to treat immune deficiencies associated with severe combined immunodeficiency (SCID) include γC, JAK3, IL7RA, RAG1, RAG2, DCLRE1C, PRKDC, LIG4, NHEJ1, CD3D, CD3E, CD3Z, CD3G, PTPRC, ZAP70, LCK, AK2, ADA, PNP, WHN, CHD7, ORAI1, STIM1, COROIA, CIITA, RFXANK, RFXS, RFXAP, RMRP, DKC1, TERT, TINF2, DCLRE1B, and SLC46A1; FANC family genes including FancA, FancB, FancC, FancD1 (BRCA2), FancD2, FancE, FancF, FancG, FancI, FancJ (BRIP1), FancL, FancM, FancN (PALB2), FancO (RAD51C), FancP (SLX4), FancQ (ERCC4), FancR (RAD51), FancS (BRCA1), FancT (UBE2T), FancU (XRCC2), FancV (MAD2L2), and FancW (RFWD3); soluble CD40; CTLA; Fas L; antibodies to CD4, CD5, CD7, CD52; antibodies to IL1, IL2, IL6; an antibody to TCR specifically present on autoreactive T cells; IL4; IL10; IL12; IL13; ILIRa, sIL1RI, sIL1RII; sTNFRI; sTNFRII; antibodies to TNF; P53, PTPN22, and DRB1^{∗}1501/DQB1^{∗}0602; globin family genes; WAS; phox; dystrophin; pyruvate kinase; CLN3; ABCD1; arylsulfatase A; SFTPB; SFTPC; NLX2.1; ABCA3; GATA1; ribosomal protein genes; TERT; TERC; DKC1; TINF2; CFTR; LRRK2; PARK2; PARK7; PINK1; SNCA; PSEN1; PSEN2; APP; SOD1; TDP43; FUS; ubiquilin 2; and/or C9ORF72.

Because most individuals lack a matched donor for BMT or non-autologous gene therapy, haploidentical parental bone marrow depleted of mature T cells is often used; however, complications include graft versus host disease (GVHD), failure to make adequate antibodies hence requiring long-term immunoglobulin replacement, late loss of T cells due to failure to engraft hematopoietic stem and progenitor cells (HSPCs), chronic warts, and lymphocyte dysregulation.

Fanconi anemia (FA) is an inherited blood disorder that leads to bone marrow failure. It is characterized, in part, by a deficient DNA-repair mechanism. At least 20% of patients with FA develop cancers such as acute myeloid leukemias, and cancers of the skin, liver, gastrointestinal tract, and gynecological systems. The skin and gastrointestinal tumors are usually squamous cell carcinomas. The average age of patients who develop cancer is 15 years for leukemia, 16 years for liver tumors, and 23 years for other tumors.

Cells from FA patients display a characteristic hypersensitivity to agents that produce interstrand DNA crosslinks such as mitomycin C or diepoxybutane. FA genes define a multicomponent pathway involved in cellular responses to DNA cross-links. Five of the FA genes (FANCA, FANCC, FANCE, FANCF and FANCG) have been cloned and the FANCA, FANCC and FANCG proteins have been shown to form a molecular complex with primarily nuclear localization. A number of mutations in the FANCC gene have been identified which are correlated with FA of differing degrees of severity.

An alternative therapeutic approach to BMT and non-autologous gene therapy in immune and blood disorder failures is *ex vivo* HSPC gene therapy, where blood or bone marrow derived HSPCs are enriched from patients, transduced with viral vectors to deliver a functional therapeutic gene (e.g., a γC gene for SCID-X1 or a FancA gene for FA), and transplanted back to the patient. The first generation *ex vivo* gene therapy for SCID-X1 used murine leukemia virus-based gamma retroviral (RV) delivery and showed significant long-term clinical improvement in treated patients. However, 5/20 patients unexpectedly developed T cell leukemia, resulting in the death of one patient. These findings precipitated intense interest in utilization of self-inactivating (SIN) viral vectors and SIN-lentiviral vectors (LV) as alternative vector platforms. While SIN-RV and SIN-LV are currently used in the clinical setting with considerable success, *ex vivo* gene therapy still faces multiple challenges that include the: 1) extensive *ex vivo* manipulation of HSPCs required to prepare them for therapeutic use that results in loss of multipotency potential and/or reduced fitness for engraftment following transplantation, 2) various conditioning regimens used to enhance engraftment of gene modified HSPCs add considerable genotoxic risks to patients, and 3) requirement of advanced infrastructures for the collection, culture, transduction, validation, and re-infusion of HSPCs, consequently restricting this form of treatment to a select few institutions worldwide.

Accordingly, compositions and methods of use include administering to a subject the pharmaceutical compositions embodied herein and/or administration of a vector encoding IL-2, for example, SEQ ID NOS: 1 and/or 2. In certain embodiments, the IL-2 therapy is conducted in combination with one or more gene therapies, *e*.*g*. expression of a therapeutic agent or a wild-type protein in cases where the expression of that particular protein is the cause of the disease. The method further comprises administration of mobilization factors such as chemokines, G-CSF, granulocyte macrophage colony stimulating factor (GM-CSF), AMD3100 and SCF. G-CSF is a cytokine whose functions in HSPC mobilization can include the promotion of granulocyte expansion and both protease-dependent and independent attenuation of adhesion molecules and disruption of the SDF-1/CXCR4 axis. Any commercially available form of G-CSF known to one of ordinary skill in the art can be used in the methods and formulations as disclosed herein, for example, Filgrastim (NEUPOGEN^{™}, Amgen Inc., Thousand Oaks, Calif.) and PEGylated Filgrastim (Pegfilgrastim, NEULASTA^{™}, Amgen Inc., Thousand Oaks, Calif.).

AMD3100 (MOZOBIL^{™}, PLERIXAFOR^{™}; Sanofi-Aventis, Paris, France), a synthetic organic molecule of the bicyclam class, is a chemokine receptor antagonist and reversibly inhibits SDF-1 binding to CXCR4, promoting HSPC mobilization. AMD3100 is approved to be used in combination with G-CSF for HSPC mobilization in patients with myeloma and lymphoma.

Gene therapies can also be utilized in immunotherapies. Accordingly, in certain embodiments, a method of treating a subject in need of immunotherapy, comprises administering to the subject:(i) an expression vector encoding SEQ ID NO: 1; and/or, (ii) a cell comprising an expression vector encoding SEQ ID NO: 1. Such subjects in need of an immunotherapeutic treatment include those suffering from cancer, immunodeficiency, immunosuppression, viral infections or combinations thereof. In certain embodiments, the method can include administration of IL-2, *e.g.* SEQ ID NO: 1, SEQ ID NO: 2 or the combination thereof.

In certain embodiments, the expression of IL-2 can be combined with expression of other cytokines, proteins and the like. See, for example, Colombo, F. et al. Combined HSV-TK/IL-2 gene therapy in patients with recurrent glioblastoma multiforme: biological and clinical results. Cancer Gene Ther 12, 835-848 (2005). doi.org/10.1038/sj.cgt. 7700851. Robert E. Sobol et al. Interleukin 2 Gene Therapy of Colorectal Carcinoma with Autologous Irradiated Tumor Cells and Genetically Engineered Fibroblasts: A Phase I Study. Clin Cancer Res September 1 1999 (5) (9) 2359-2365. Philip A. Durost, et al. Human Gene Therapy. Mar 2018.352-365.http://doi.org/10.1089/hum.2017.072. Kevin S. Goudy et al. Inducible Adeno-Associated Virus-Mediated IL-2 Gene Therapy Prevents Autoimmune Diabetes. The Journal of Immunology March 15, 2011, 186 (6) 3779-3786; DOI: 10.4049/jimmunol. 1001422. Mahzad Akbarpour et al., Insulin B chain 9-23 gene transfer to hepatocytes protects from type 1 diabetes by inducing Ag-specific FoxP3+ Tregs. Science Translational Medicine. 7, 289ra81 (2015). DOI: 10.1126/scitranslmed.aaa3032.

Any type of cell which can be transformed with a vector expressing IL-2, for example, SEQ ID NOS: 1, 2 or the combination thereof. Examples include autologous cells, allogeneic cells, haplotype matched cells, haplotype mismatched cells, haplo-identical cells, xenogeneic cells, cell lines or combinations thereof. The types of cells, include for example, stem cells, cord blood cells, adult stem cells, mesenchymal stem cells, mesenchymal stromal cells, induced pluripotent stem cells, autologous stem cells, bone marrow cells, hematopoietic cells, hematopoietic stem cells, somatic cells, germ line cells, differentiated cells, somatic stem cells, embryonic stem cells or combinations thereof.

Treatment using *in vivo* gene therapy, which includes the direct delivery of a viral vector to a patient, is a simple and attractive approach because it may not require any genotoxic conditioning (or could require less genotoxic conditioning) nor *ex vivo* cell processing and thus could be adopted at many institutions worldwide, including those in developing countries, as the therapy could be administered through an injection, similar to what is already done worldwide for the delivery of vaccines.

Foamy virus (FV) vectors are non-pathogenic integrating retroviruses, which are highly effective for HSPC gene therapy and potentially safer than SIN-RV and LV. For example, foamy vector proviruses integrate less frequently in genes than LV vectors, and have a reduced propensity to transactivate nearby genes than LV or RV vectors. These properties likely contribute to their safety as established in the canine model and in the murine xenotransplantation model. Unlike VSV-G pseudotyped LV vectors, FV vectors are resistant to human serum inactivation, which gives them a specific advantage during *in vivo* delivery and would allow for multiple infusions of the same FV vector if multiple dosages were required.

Other vectors include, for example, Adeno-associated virus (AAV) vectors . These vectors are considered to have the best safety and efficacy profile for the delivery of genes in humans *in vivo.* Therefore AAV vectors have been extensively used for *in vivo* gene therapy and have been shown safe and effective in pre-clinical models as well as in clinical trials. AAV vectors have been successful in phase HI studies for hemophilia B, cystic fibrosis, alpha-1 anti-trypsin deficiency, Parkinson disease, Duchenne muscular dystrophy and Leber's congenital amaurosis (Selot et al., Current Pharmaceutical Biotechnology, 2013, 14,1072-1082). Alipogene tiparvovec (GLYBERA^{™}, uniQure) has been granted marketing authorization in Europe as a gene therapy for the treatment of lipoprotein lipase deficiency (LPLD).

### Methods for Isolation of Cells

Any number of methods known in the art can be used to isolate cells, such as T cells, or any other cell type that may be used in carrying out the treatment of a subject. Thus, also provided are various other genetically engineered cells expressing the chimeric antigen receptors e.g., CARs. The cells generally are eukaryotic cells, such as mammalian cells, and typically are human cells. In some embodiments, the cells are derived from the blood, bone marrow, lymph, or lymphoid organs, are cells of the immune system, such as cells of the innate or adaptive immunity, e.g. myeloid or lymphoid cells, including lymphocytes, typically T cells and/or NK cells. Other exemplary cells include stem cells, such as multipotent and pluripotent stem cells, including induced pluripotent stem cells (iPSCs). The cells typically are primary cells, such as those isolated directly from a subject and/or isolated from a subject and frozen. In some embodiments, the cells include one or more subsets of T cells or other cell types, such as whole T cell populations, CD4⁺ cells, CD8⁺ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. Among the methods include off-the-shelf methods. In some aspects, such as for off-the-shelf technologies, the cells are pluripotent and/or multipotent, such as stem cells, such as induced pluripotent stem cells (iPSCs). In some embodiments, the methods include isolating cells from the subject, preparing, processing, culturing, and/or engineering them, as described herein, and reintroducing them into the same patient, before or after cryopreservation.

Among the sub-types and subpopulations of T cells and/or of CD4⁺ and/or of CD8⁺ T cells are naive T (T_{N}) cells, effector T cells (T_{EFF}), memory T cells and sub-types thereof, such as stem cell memory T (T_{SCMX} central memory T (T_{CM} effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as T_{H}1 cells, T_{H}2 cells, T_{H}3 cells, T_{H}17 cells, T_{H}9 cells, T_{H}22 cells, follicular helper T cells, alpha/beta T (αβ T) cells, and delta/gamma T (yδ T) cells.

In certain embodiments, the T cell is a mammalian regulatory T cell (Treg), wherein the Treg cell is CD4⁺, CD25⁺, CD127⁻, FOXP3⁺ and/or Helios^{+/-}. In other embodiments, the T cell is a mammalian regulatory T cell (Treg), wherein the Treg cell is CD4⁺, CD25⁺, CD127⁻, and/or FOXP3⁺.

In some embodiments, the cells are natural killer (NK) cells. In some embodiments, the cells are monocytes or granulocytes, e.g., myeloid cells, macrophages, neutrophils, dendritic cells, mast cells, eosinophils, and/or basophils.

In some embodiments, the cells include one or more nucleic acids introduced via genetic engineering, and thereby express recombinant or genetically engineered products of such nucleic acids. In some embodiments, the nucleic acids are heterologous, i.e., normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature, including one comprising chimeric combinations of nucleic acids encoding various domains from multiple different cell types.

In some embodiments, preparation of the engineered cells includes one or more culture and/or preparation steps. The cells for introduction of the CAR, may be isolated from a sample, such as a biological sample, e.g., one obtained from or derived from a subject. In some embodiments, the subject from which the cell is isolated is one having the disease or condition or in need of a cell therapy or to which cell therapy will be administered. The subject in some embodiments is a human in need of a particular therapeutic intervention, such as the adoptive cell therapy for which cells are being isolated, processed, and/or engineered.

Accordingly, the cells in some embodiments are primary cells, e.g., primary human cells. The samples include tissue, fluid, and other samples taken directly from the subject, as well as samples resulting from one or more processing steps, such as separation, centrifugation, genetic engineering (e.g. transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample from which the cells are derived or isolated is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some embodiments, the cells are derived from cell lines, e.g., T cell lines. The cells in some embodiments are obtained from a xenogeneic source, for example, from mouse, rat, non-human primate, or pig.

In some embodiments, isolation of the cells includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components.

In some examples, cells from the circulating blood of a subject are obtained, e.g., by apheresis or leukapheresis. The samples, in some aspects, contain lymphocytes, including T cells, monocytes, granulocytes, B cells, other nucleated white blood cells, red blood cells, and/or platelets, and in some aspects contains cells other than red blood cells and platelets.

In some embodiments, the blood cells collected from the subject are washed, e.g., to remove the plasma fraction and to place the cells in an appropriate buffer or media for subsequent processing steps. In some embodiments, the cells are washed with phosphate buffered saline (PBS). In some embodiments, the wash solution lacks calcium and/or magnesium and/or many or all divalent cations. In some aspects, a washing step is accomplished a semi-automated "flow-through" centrifuge (for example, the Cobe 2991 cell processor, Baxter) according to the manufacturer's instructions. In some aspects, a washing step is accomplished by tangential flow filtration (TFF) according to the manufacturer's instructions. In some embodiments, the cells are resuspended in a variety of biocompatible buffers after washing, such as, for example, Ca⁺⁺/Mg⁺⁺ free PBS. In certain embodiments, components of a blood cell sample are removed and the cells directly resuspended in culture media.

In some embodiments, the methods include density-based cell separation methods, such as the preparation of white blood cells from peripheral blood by lysing the red blood cells and centrifugation through a Percoll or Ficoll gradient.

In some embodiments, the isolation methods include the separation of different cell types based on the expression or presence in the cell of one or more specific molecules, such as surface markers, e.g., surface proteins, intracellular markers, or nucleic acid. In some embodiments, any known method for separation based on such markers may be used. In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100% enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types. T cells, are isolated by positive or negative selection techniques. For example, CD3⁺, CD28⁺ T cells can be positively selected using anti-CD3/anti-CD28 conjugated magnetic beads (e.g., DYNABEADS^{®} M-450 CD3/CD28 T Cell Expander).

In some embodiments, isolation is carried out by enrichment for a particular cell population by positive selection, or depletion of a particular cell population, by negative selection. In some embodiments, positive or negative selection is accomplished by incubating cells with one or more antibodies or other binding agent that specifically bind to one or more surface markers expressed or expressed (marker" 1") at a relatively higher level (marker^{hlgh}) on the positively or negatively selected cells, respectively.

In some embodiments, T cells are separated from a PBMC sample by negative selection of markers expressed on non-T cells, such as B cells, monocytes, or other white blood cells, such as CD 14. In some aspects, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

In some embodiments, CD8⁺ cells are further enriched for or depleted of naive, central memory, effector memory, and/or central memory stem cells, such as by positive or negative selection based on surface antigens associated with the respective subpopulation. In some embodiments, enrichment for central memory T (T_{CM}) cells is carried out to increase efficacy, such as to improve long-term survival, expansion, and/or engraftment following administration, which in some aspects is particularly robust in such sub-populations. See Terakura et al. (2012) Blood. 1:72-82; Wang et al. (2012) J Immunother. 35(9):689-701. In some embodiments, combining T_{CM}-enriched CD8⁺ T cells and CD4⁺ T cells further enhances efficacy.

In some embodiments, the enrichment for central memory T (T_{CM}) cells is based on positive or high surface expression of CD45RO, CD62L, CCR7, CD28, CD3, and/or CD 127; in some aspects, it is based on negative selection for cells expressing or highly expressing CD45RA and/or granzyme B.

In some aspects, a CD4 expression-based selection step is used to generate the CD4⁺ cell population or sub-population, such that both the positive and negative fractions from the CD4-based separation are retained and used in subsequent steps of the methods, optionally following one or more further positive or negative selection steps.

In one example, a sample of PBMCs or other white blood cell sample is subjected to selection of CD4⁺ cells, where both the negative and positive fractions are retained. The negative fraction then is subjected to negative selection based on expression of, for example, CD 14 and CD45RA, and positive selection based on a marker characteristic of central memory T cells, such as CD62L or CCR7, where the positive and negative selections are carried out in either order.

CD4⁺ T helper cells are sorted into naive, central memory, and effector cells by identifying cell populations that have cell surface antigens. CD4⁺ lymphocytes can be obtained by standard methods. In some embodiments, naive CD4⁺ T lymphocytes are CD45RO⁺, CD45RA⁺, CD62L⁺, CD4⁺ T cells. In some embodiments, central memory CD4⁺ cells are CD62L⁺ and CD45RO⁺.

In one example, to enrich for CD4⁺ cells by negative selection, a monoclonal antibody cocktail typically includes antibodies to CD14, CD20, CD11b, CD16, HLA-DR, and CD8. In some embodiments, the antibody or binding partner is bound to a solid support or matrix, such as a magnetic bead or paramagnetic bead, to allow for separation of cells for positive and/or negative selection. For example, in some embodiments, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In vitro and In vivo, p 17-25 Edited by: S. A. Brooks and U. Schumacher © Humana Press Inc., Totowa, NJ).

In some aspects, the sample or composition of cells to be separated is incubated with small, magnetizable or magnetically responsive material, such as magnetically responsive particles or microparticles, such as paramagnetic beads (e.g., such as DYNABEADS or MACS beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some embodiments, the magnetic particle or bead comprises a magnetically responsive material bound to a specific binding member, such as an antibody or other binding partner. There are many well-known magnetically responsive materials used in magnetic separation methods. Suitable magnetic particles include those described in Molday, U.S. Pat. No. 4,452,773, and in European Patent Specification EP 452342 B, which are hereby incorporated by reference. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In certain embodiments, the magnetically responsive particles are coated in primary antibodies or other binding partners, secondary antibodies, lectins, enzymes, or streptavidin. In certain embodiments, the magnetic particles are attached to cells via a coating of primary antibodies specific for one or more markers. In certain embodiments, the cells, rather than the beads, are labeled with a primary antibody or binding partner, and then cell-type specific secondary antibody- or other binding partner (e.g., streptavidin)-coated magnetic particles, are added. In certain embodiments, streptavidin-coated magnetic particles are used in conjunction with biotinylated primary or secondary antibodies.

In some embodiments, the magnetically responsive particles are left attached to the cells that are to be subsequently incubated, cultured and/or engineered; in some aspects, the particles are left attached to the cells for administration to a patient. In some embodiments, the magnetizable or magnetically responsive particles are removed from the cells. Methods for removing magnetizable particles from cells are known and include, e.g., the use of competing non-labeled antibodies, magnetizable particles or antibodies conjugated to cleavable linkers, etc. In some embodiments, the magnetizable particles are biodegradable.

In some embodiments, the affinity-based selection is via magnetic-activated cell sorting (MACS) (Miltenyi Biotec, Auburn, CA). Magnetic Activated Cell Sorting (MACS) systems are capable of high-purity selection of cells having magnetized particles attached thereto. In certain embodiments, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain embodiments, the non-target cells are labelled and depleted from the heterogeneous population of cells.

In certain embodiments, the isolation or separation is carried out using a system, device, or apparatus that carries out one or more of the isolation, cell preparation, separation, processing, incubation, culture, and/or formulation steps of the methods. In some aspects, the system is used to carry out each of these steps in a closed or sterile environment, for example, to minimize error, user handling and/or contamination. In one example, the system is a system as described in International Patent Application, Publication Number WO2009/072003, or US 20110003380A1.

In some embodiments, the system or apparatus carries out one or more, e.g., all, of the isolation, processing, engineering, and formulation steps in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some aspects, the separation and/or other steps is carried out using CliniMACS system (Miltenyi Biotec), for example, for automated separation of cells on a clinical-scale level in a closed and sterile system. Components can include an integrated microcomputer, magnetic separation unit, peristaltic pump, and various pinch valves. The integrated computer in some aspects controls all components of the instrument and directs the system to perform repeated procedures in a standardized sequence. The magnetic separation unit in some aspects includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some aspects uses antibody-coupled magnetizable particles that are supplied in a sterile, non-pyrogenic solution. In some embodiments, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some embodiments, the cell populations for use with the methods described herein are unlabeled and are not retained in the column. In some embodiments, the cell populations for use with the methods described herein are labeled and are retained in the column. In some embodiments, the cell populations for use with the methods described herein are eluted from the column after removal of the magnetic field, and are collected within the cell collection bag.

In certain embodiments, separation and/or other steps are carried out using the CliniMACS Prodigy system (Miltenyi Biotec). The CliniMACS Prodigy system in some aspects is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood may be automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. (2012) J Immunother. 35(9): 651-660, Terakura et al. (2012) Blood. 1:72-82, and Wang et al. (2012) Immunother. 35(9):689-701.

In some embodiments, a cell population described herein is collected and enriched (or depleted) via flow cytometry, in which cells stained for multiple cell surface markers are carried in a fluidic stream. In some embodiments, a cell population described herein is collected and enriched (or depleted) via preparative scale (FACS)-sorting. In certain embodiments, a cell population described herein is collected and enriched (or depleted) by use of microelectromechanical systems (MEMS) chips in combination with a FACS-based detection system (see, e.g., WO 2010/033140, Cho et al. (2010) Lab Chip 10, 1567-1573; and Godin et al. (2008) J Biophoton. 1(5):355-376. In both cases, cells can be labeled with multiple markers, allowing for the isolation of well-defined T cell subsets at high purity.

In some embodiments, the antibodies or binding partners are labeled with one or more detectable marker, to facilitate separation for positive and/or negative selection. For example, separation may be based on binding to fluorescently labeled antibodies. In some examples, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flow-cytometric detection system. Such methods allow for positive and negative selection based on multiple markers simultaneously.

In some embodiments, the preparation methods include steps for freezing, e.g., cryopreserving, the cells, either before or after isolation, incubation, and/or engineering. In some embodiments, the freeze and subsequent thaw step removes granulocytes and, to some extent, monocytes in the cell population. In some embodiments, the cells are suspended in a freezing solution, e.g., following a washing step to remove plasma and platelets. Any of a variety of known freezing solutions and parameters in some aspects may be used. One example involves using PBS containing 20% DMSO and 8% human serum albumin (HSA), or other suitable cell freezing media. This is then diluted 1 : 1 with media so that the final concentration of DMSO and HSA are 10% and 4%, respectively. The cells are then frozen to -80° C. at a rate of 1° per minute and stored in the vapor phase of a liquid nitrogen storage tank.

In some embodiments, the provided methods include cultivation, incubation, culture, and/or genetic engineering steps. For example, in some embodiments, provided are methods for incubating and/or engineering the depleted cell populations and culture-initiating compositions.

Thus, in some embodiments, the cell populations are incubated in a culture-initiating composition. The incubation and/or engineering may be carried out in a culture vessel, such as a unit, chamber, well, column, tube, tubing set, valve, vial, culture dish, bag, or other container for culture or cultivating cells.

In some embodiments, the cells are incubated and/or cultured prior to or in connection with genetic engineering. The incubation steps can include culture, cultivation, stimulation, activation, and/or propagation. In some embodiments, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some embodiments, the stimulating conditions or agents include one or more agent, e.g., ligand, which is capable of activating an intracellular signaling domain of a TCR complex. In some aspects, the agent turns on or initiates TCR/CD3 intracellular signaling cascade in a T cell. Such agents can include antibodies, such as those specific for a TCR, e.g. anti-CD3. In some embodiments, the stimulating conditions include one or more agent, e.g. ligand, which is capable of stimulating a costimulatory receptor, e.g., anti-CD28. In some embodiments, such agents and/or ligands may be, bound to solid support such as a bead, and/or one or more cytokines. Optionally, the expansion method may further comprise the step of adding anti-CD3 and/or anti CD28 antibody to the culture medium (e.g., at a concentration of at least about 0.5 ng/ml). In some embodiments, the stimulating agents include IL-2, IL-15 and/or IL-7. In some aspects, the IL-2, *e.g.* SEQ ID NO: 1, concentration is at least about 10 units/mL.

In some aspects, incubation is carried out in accordance with techniques such as those described in US Patent No. 6,040,1 77 to Riddell et al.; Klebanoff et al. (2012) J Immunother. 35(9): 651-660, Terakura et al. (2012) Blood. 1:72-82, and/or Wang et al. (2012) J Immunother. 35(9):689-701.

In some embodiments, the T cells are expanded by adding to the culture-initiating composition feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g. for a time sufficient to expand the numbers of T cells). In some aspects, the non-dividing feeder cells can comprise gamma-irradiated PBMC feeder cells. In some embodiments, the PBMC are irradiated with gamma rays in the range of about 3000 to 3600 rads to prevent cell division. In some aspects, the feeder cells are added to culture medium prior to the addition of the populations of T cells.

In some embodiments, the stimulating conditions include temperature suitable for the growth of human T lymphocytes, for example, at least about 25 degrees Celsius, generally at least about 30 degrees, and generally at or about 37 degrees Celsius. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some aspects is provided in any suitable amount, such as a ratio of LCL feeder cells to initial T lymphocytes of at least about 10: 1.

### Pharmaceutical Compositions

The IL-2, *e.g.* SEQ ID NO: 1, may be formulated as pharmaceutical compositions with pharmaceutically acceptable carriers. The compounds are administered to the subject in an effective amount for treating cancer. An "effective amount", for instance, is an amount necessary or sufficient to realize a desired biologic effect. An effective amount for treating cancer, for instance, could be that amount necessary to (i) prevent or slow further growth of a cancer and/or (ii) kill existing cancer cells. According to some aspects of the invention, an effective amount is that amount of a compound of the invention alone or in combination with another medicament, which when combined or co-administered or administered alone, results in a therapeutic response to the disease, either in the prevention or the treatment of the disease. The biological effect may be the amelioration and or absolute elimination of the cancer. In another embodiment, the biological effect is the complete abrogation of the disease, as evidenced for example, by the absence of a symptom of the disease e.g. renal cell carcinoma.

The effective amount of a compound of the invention in the treatment of a disease described herein may vary depending upon the specific compound used, the mode of delivery of the compound, and whether it is used alone or in combination. The effective amount for any particular application can also vary depending on such factors as the disease being treated, the particular compound being administered, the size of the subject, or the severity of the disease or condition. One of ordinary skill in the art can empirically determine the effective amount of a particular molecule of the invention without necessitating undue experimentation. Combined with the teachings provided herein, by choosing among the various active compounds and weighing factors such as potency, relative bioavailability, patient body weight, severity of adverse side-effects and preferred mode of administration, an effective prophylactic or therapeutic treatment regimen can be planned which does not cause substantial toxicity and yet is entirely effective to treat the particular subject.

In certain embodiments, a pharmaceutical composition comprises IL-2 in a range from about 0.001 mg/ml to 3 mg/ml, SDS in a range of about 0.0001 mg/ml to about 1 mg/ml, a non-ionic osmolytes, a pH 7-8 phosphate buffer or combinations thereof. In certain embodiments, a pharmaceutical composition consists of IL-2 in a range from about 0.001 mg to 3 mg/mL, SDS in a range of about 0.0001 mg/ml to about 10 mg/ml, a non-ionic osmolytes and a pH 7-8 phosphate buffer. In certain embodiments, an osmolyte comprises mannitol, sorbitol, xylitol or a pH 7-8 phosphate buffer.

Subject doses of the compounds described herein typically range from about 0.1 µg to 10,000 mg, or from about 1 µg/day to 8000 mg, or from about 10 µg to 100 µg. Stated in terms of subject body weight, typical dosages range from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 5 mg/kg/body weight to about 100 mg/kg/body weight, about 5 microgram/kg/body weight to about 10,000 milligram/kg/body weight, etc., can be administered, based on the numbers described above.

In certain embodiments, exemplary effective doses of the IL-2 molecules embodied herein, *e.g.* SEQ ID NO: 1, include between 0.1 µg/kg and 100 mg/kg body weight, e.g., 0.1, 0.2, 0.3, 0.4, 0.5, 0.6, 0.7, 0.8, 0.9, 1, 2, 3, 4, 5, 6, 7, 8, 9, 10, 15, 20, 25, 30, 35, 40, 45, 50, 55, 60, 65, 70, 75, 80, 85, 90, 95, 100, 200, 300, 400, 500, 600, 700, 800, or 900 µg/kg body weight or 1, 2, 3, 4, 5, 6, 7, 8, 9, 10, 20, 30, 40, 50, 60, 70, 80, 90, or 100 mg/kg body weight.

In some cases, the IL-2 is administered daily, e.g., every 24 hours. Or, the IL-2 is administered continuously or several times per day, e.g., every 1 hour, every 2 hours, every 3 hours, every 4 hours, every 5 hours, every 6 hours, every 7 hours, every 8 hours, every 9 hours, every 10 hours, every 11 hours, or every 12 hours.

Exemplary effective daily doses of IL-2, *e.g.* SEQ ID NO: 1, include between 0.1 µg/kg and 100 µg/kg body weight, e.g., 0.1, 0.3, 0.5, 1, 5, 10, 15, 20, 25, 30, 35, 40, 45, 50, 55, 60, 65, 70, 75, 80, 85, 90, 95, or 99 µg/kg body weight.

Alternatively, the IL-2, *e.g.* SEQ ID NO: 1, is administered about once per week, e.g., about once every 7 days. Or, the IL-2 is administered twice per week, three times per week, four times per week, five times per week, six times per week, or seven times per week. Exemplary effective weekly doses of IL-2 include between 0.0001 mg/kg and 4 mg/kg body weight, e.g., 0.001, 0.003, 0.005, 0.01. 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, or 4 mg/kg body weight. For example, an effective weekly dose of IL-2, *e.g.* SEQ ID NO: 1, is between 0.001 µg/kg body weight and 400 µg/kg body weight. Alternatively, IL-2, *e.g.* SEQ ID NO: 1, is administered at a fixed dose or based on body surface area (i.e., per m²).

In some cases, subjects receive two 6-week cycles consisting of 4 weekly IL-2, *e.g.* SEQ ID NO: 1, intravenous doses followed by a 2-week rest period. Ultimately, the attending physician or veterinarian decides the appropriate amount and dosage regimen.

The absolute amount will depend upon a variety of factors including the concurrent treatment, the number of doses and the individual patient parameters including age, physical condition, size and weight. These are factors well known to those of ordinary skill in the art and can be addressed with no more than routine experimentation. It is preferred generally that a maximum dose be used, that is, the highest safe dose according to sound medical judgment.

In certain embodiments, the compositions described herein are administered systemically, intravenously, subcutaneously, intramuscularly, intraperitoneally, intravesically, or by instillation. The inhibitors and the aldesleukin may be administered simultaneously or sequentially.

### Combination Therapies

Optionally, the IL-2 pharmaceutical compositions embodied herein are administered in combination with any other standard therapy; such methods are known to the skilled artisan and described in Remington's Pharmaceutical Sciences by E. W. Martin. If desired, the compositions are administered in combination with any conventional anti-neoplastic therapy, including but not limited to, immunotherapy, therapeutic antibodies, targeted therapy, surgery, radiation therapy, chemotherapy, a cytokine therapy, a T-cell therapy, an NK therapy, an immune system checkpoint inhibitor, immunostimulating substances, gene therapy, antibodies and/or dendritic cells.

In certain embodiments, the administration of a pharmaceutical composition comprising IL-2 in a range from about 0.001 mg/ml to 3 mg/ml, SDS in a range of about 0.0001 mg/ml to about 10 mg/ml, a non-ionic osmolytes, a pH 7-8 phosphate buffer or combinations thereof, is administered with one or more secondary active agents. For example, in the treatment of cancer, the pharmaceutical composition comprising the IL-2 molecule embodied herein is administered with a chemotherapeutic agent. Chemotherapeutic drugs include alkylating agents (e.g., platinum-based drugs, tetrazines, aziridines, nitrosoureas, nitrogen mustards), anti-metabolites (e.g., anti-folates, fluoropyrimidines, deoxynucleoside analogues, thiopurines), anti-microtubule agents (e.g., vinca alkaloids, taxanes), topoisomerase inhibitors (e.g., topoisomerase I and II inhibitors), cytotoxic antibiotics (e.g., anthracyclines) and immunomodulatory drugs (e.g., thalidomide and analogs).

Accordingly, in certain embodiments, the IL-2 pharmaceutical compositions embodied herein are administered as a combination therapy with one or more chemotherapeutic agents. A "chemotherapeutic agent" is a chemical compound useful in the treatment of cancer. Examples of chemotherapeutic agents include mitomycin C, Erlotinib (TARCEVA^{™}, Genentech/OSI Pharm.), Bortezomib (VELCADE^{™}, Millennium Pharm.), Fulvestrant (FASLODEX^{™}, Astrazeneca), Sutent (SU11248, Pfizer), Letrozole (FEMARA^{™}, Novartis), Imatinib mesylate (GLEEVEC^{™}, Novartis), PTK787/ZK 222584 (Novartis), Oxaliplatin (ELOXATIN^{™}, Sanofi), 5-FU (5-fluorouracil), Leucovorin, Rapamycin (Sirolimus, RAPAMUNE^{™}, Wyeth), Lapatinib (GSK572016, GlaxoSmithKline), Lonafarnib (SCH 66336), Sorafenib (BAY43-9006, Bayer Labs.), and Gefitinib (IRESSA^{™}, Astrazeneca), AG1478, AG1571 (SU 5271; Sugen), alkylating agents such as Thiotepa and CYTOXAN^{™} cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including the synthetic analogue topotecan); bryostatin; callystatin; CC-1065 (including its adozcicsin, carzcicsin and bizcicsin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin γ1 and calicheamicin omega 1 (Angew Chem. Intl. Ed. Engl. (1994) 33:183-186); dynemicin, including dynemicin A; bisphosphonates, such as clodronate; an esperamicin; as well as neocarzinostatin chromophore and related chromoprotein enediyne antibiotic chromophores), aclacinomysins, actinomycin, anthramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN^{™} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, strcptonigrin, strcptozocin, tubcrcidin, ubenimcx, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacytidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{™} polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosinc; arabinoside ("Ara-C"); cyclophosphamidc; thiotcpa; taxoids, e.g., TAXOL^{™} paclitaxel (Bristol-Myers Squibb Oncology, Princeton, N.J.), ABRAXANE^{™} Cremophor-free, albumin-engineered nanoparticle formulation of paclitaxel (American Pharmaceutical Partners, Schaumberg, 111.), and TAXOTERE^{™} doxetaxel (Rhone-Poulenc Rorer, Antony, France); chloranbucil; GEMZAR^{™} gemcitabine; 6-thioguanine; mercaptopurine; methotrexate; platinum analogs such as cisplatin and carboplatin; vinblastine; platinum; etoposide (VP-16); ifosfamide; mitoxantrone; vincristine; NAVELBINE^{™} vinorelbine; novantrone; teniposide; edatrexate; daunomycin; aminopterin; xeloda; ibandronate; CPT-11; topoisomerase inhibitor RFS 2000; difluoromethylornithine (DMFO); retinoids such as retinoic acid; capecitabine; and pharmaceutically acceptable salts, acids or derivatives of any of the above.

Also included in this definition of "chemotherapeutic agent" are: (i) anti-hormonal agents that act to regulate or inhibit hormone action on tumors such as anti-estrogens and selective estrogen receptor modulators (SERMs), including, for example, tamoxifen (including NOLVADEX^{™} (tamoxifen)), raloxifene, droloxifene, 4-hydroxytamoxifen, trioxifene, keoxifene, LY117018, onapristone, and FARESTON^{™} (toremifene); (ii) aromatase inhibitors that inhibit the enzyme aromatase, which regulates estrogen production in the adrenal glands, such as, for example, 4(5)-imidazoles, aminoglutethimide, MEGASE^{™} (megestrol acetate), AROMASIN^{™} (exemestane), formestanie, fadrozole, RIVISOR^{™} (vorozole), FEMARA^{™} (letrozole), and ARIMIDEX^{™} (anastrozole); (iii) anti-androgens such as flutamide, nilutamide, bicalutamide, leuprolide, and goserelin; as well as troxacitabine (a 1,3-dioxolane nucleoside cytosine analog); (iv) aromatase inhibitors; (v) protein kinase inhibitors; (vi) lipid kinase inhibitors; (vii) antisense oligonucleotides, particularly those which inhibit expression of genes in signaling pathways implicated in aberrant cell proliferation, such as, for example, PKC-alpha, Ralf and H-Ras; (viii) ribozymes such as a VEGF expression inhibitor (e.g., ANGIOZYME^{™} (ribozyme)) and a HER2 expression inhibitor; (ix) vaccines such as gene therapy vaccines, for example, ALLOVECTIN^{™} vaccine, LEUVECTIN^{™} vaccine, and VAXID^{™} vaccine; PROLEUKIN^{™} rIL-2; LURTOTECAN^{™} topoisomerase 1 inhibitor; ABARELIX^{™} rmRH; (x) anti-angiogenic agents such as bevacizumab (AVASTIN^{™}, Genentech); and (xi) pharmaceutically acceptable salts, acids or derivatives of any of the above.

In certain embodiments, the IL-2 pharmaceutical compositions embodied herein are administered as a combination therapy with one or more anti-inflammatory agents. Anti-inflammatory agents include steroidal and non-steroidal anti-inflammatory drugs with analgesic, antipyretic and anti-inflammatory effects. NSAIDs include non-selective inhibitors of the enzyme cyclooxygenase. Specific examples of NSAIDs include aspirin, propionic acid derivatives such as ibuprofen, fenoprofen, ketoprofen, flurbiprofen, oxaprozin and naproxen, acetic acid derivatives such as indomethacin, sulindac, etodolac, diclofenac, enolic acid derivatives such as piroxicam, meloxicam, tenoxicam, droxicam, lornoxicam and isoxicam, fenamic acid derivatives such as mefenamic acid, meclofenamic acid, flufenamic acid, tolfenamic acid, and COX-2 inhibitors such as celecoxib, etoricoxib, lumiracoxib, parecoxib, rofecoxib, rofecoxib, and valdecoxib. NSAIDs can be indicated for the symptomatic relief of conditions such as rheumatoid arthritis, osteoarthritis, inflammatory arthropathies, ankylosing spondylitis, psoriatic arthritis, Reiter's syndrome, acute gout, dysmenorrhoea, metastatic bone pain, headache and migraine, postoperative pain, mild-to-moderate pain due to inflammation and tissue injury, pyrexia, ileus, and renal colic.

In certain embodiments, the IL-2 pharmaceutical compositions embodied herein are administered as a combination therapy with one or more immune modulating agents. Immunomodulatory agents include tumor necrosis factor; interferon alpha, beta, and gamma; interleukins and other cytokines; F42K and other cytokine analogs; or MIP-1, MIP-1beta, MCP-1, RANTES, and other chemokines.

In certain embodiments, the IL-2 pharmaceutical compositions embodied herein are administered as a combination therapy with one or more immune checkpoint inhibitors.

Immune checkpoints refer to inhibitory pathways of the immune system that are responsible for maintaining self-tolerance and modulating the duration and amplitude of physiological immune responses. Certain cancer cells thrive by taking advantage of immune checkpoint pathways as a major mechanism of immune resistance, particularly with respect to T cells that are specific for tumor antigens. For example, certain cancer cells may overexpress one or more immune checkpoint proteins responsible for inhibiting a cytotoxic T cell response. Thus, immune checkpoint modulators may be administered to overcome the inhibitory signals and permit and/or augment an immune attack against cancer cells. Immune checkpoint modulators may facilitate immune cell responses against cancer cells by decreasing, inhibiting, or abrogating signaling by negative immune response regulators (e.g. CTLA4), or may stimulate or enhance signaling of positive regulators of immune response (e.g. CD28). Administration of the IL-2 pharmaceutical compositions embodied herein would therefore stimulate the immune response against, for example a tumor or viral infected cells and maintain the immune response.

Immunotherapy agents targeted to immune checkpoint modulators may be administered to encourage immune attack targeting cancer cells. Immunotherapy agents may be or include antibody agents that target (e.g., are specific for) immune checkpoint modulators. Examples of immunotherapy agents include antibody agents targeting one or more of CTLA-4, PD-1, PD-L1, GITR, OX40, LAG-3, KIR, TIM-3, CD28, CD40; and CD137. Specific examples of antibody agents may include monoclonal antibodies. Certain monoclonal antibodies targeting immune checkpoint modulators are available. For instance, ipilumimab targets CTLA-4; tremelimumab targets CTLA-4; pembrolizumab targets PD-1, etc. In some embodiments, such therapy involves administration of one or more of nivolumab (BMS-936558, MDX-1106, ONO-4538, a fully human Immunoglobulin G4 (IgG4) monoclonal PD-1 antibody), pembrolizumab (MK-3475, a humanized monoclonal IgG4 anti-PD-1 antibody), BMS-936559 (a fully human IgG4 PD-L1 antibody), MPDL3280A (a humanized engineered IgG1 monoclonal PD-L1 antibody) and/or MEDI4736 (a humanized engineered IgG1 monoclonal PD-L1 antibody).

### Pharmaceutical Therapeutics

The invention includes IL-2, *e.g.* SEQ ID NO: 1, that are useful for the treatment of cancer, autoimmunity, cell therapies, gene therapies and the like. For therapeutic uses, the IL-2, *e.g.* SEQ ID NO: 1, embodied herein may be administered systemically, for example, formulated in a pharmaceutically-acceptable buffer such as physiological saline. Preferable routes of administration include, for example, subcutaneous, intravenous, interperitoneally, intramuscular, or intradermal injections that provide continuous, sustained levels of the drug in the patient. Treatment of human patients or other animals will be carried out using a therapeutically effective amount of a therapeutic identified herein in a physiologically-acceptable carrier. Suitable carriers and their formulation are described, for example, in Remington's Pharmaceutical Sciences by E. W. Martin. The amount of the therapeutic agent to be administered varies depending upon the manner of administration, the age and body weight of the patient, and with the clinical symptoms of the cancer. Generally, amounts will be in the range of those used for other agents used in the treatment of other diseases associated with cancer, although in certain instances lower amounts will be needed because of the increased specificity of the compound.

### Formulation of Pharmaceutical Compositions

The compositions may be provided in a dosage form that is suitable for parenteral (e.g., subcutaneously, intravenously, intramuscularly, intravesicularly or intraperitoneally) administration route. An advantageous method of administration is intravenous infusion. The pharmaceutical therapeutic agent may be formulated according to conventional pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York).

In certain embodiments, a pharmaceutical composition comprises IL-2 in a range from about 0.001 mg/ml to 3 mg/ml, SDS in a range of about 0.0001 mg/ml to about 10 mg/ml, a non-ionic osmolytes, a pH 7-8 phosphate buffer or combinations thereof. In certain embodiments, a pharmaceutical composition consists of IL-2 in a range from about 0.001 mg to 3 mg/mL, SDS in a range of about 0.0001 mg/ml to about 10 mg/ml, a non-ionic osmolytes and a pH 7-8 phosphate buffer. In certain embodiments, an osmolyte comprises mannitol, sorbitol, xylitol or a pH 7-8 phosphate buffer.

Pharmaceutical compositions according to the invention may be formulated to release the IL-2, *e.g.* SEQ ID NO: 1, substantially immediately upon administration or at any predetermined time or time period after administration. The latter types of compositions are generally known as controlled release formulations, which include (i) formulations that create a substantially constant concentration of the drug within the body over an extended period of time; (ii) formulations that after a predetermined lag time create a substantially constant concentration of the drug within the body over an extended period of time; (iii) formulations that sustain action during a predetermined time period by maintaining a relatively, constant, effective level in the body with concomitant minimization of undesirable side effects associated with fluctuations in the plasma level of the active substance (sawtooth kinetic pattern); (iv) formulations that localize action by, e.g., spatial placement of a controlled release composition adjacent to or in contact with the thymus; (v) formulations that allow for convenient dosing, such that doses are administered, for example, once every one or two weeks; and (vi) formulations that cancer by using carriers or chemical derivatives to deliver the therapeutic agent to a particular cell type (e.g., neoplastic cell). For some applications, controlled release formulations obviate the need for frequent dosing during the day to sustain the plasma level at a therapeutic level.

Any of a number of strategies can be pursued in order to obtain controlled release in which the rate of release outweighs the rate of metabolism of the compound in question. In one example, controlled release is obtained by appropriate selection of various formulation parameters and ingredients, including, e.g., various types of controlled release compositions and coatings. Thus, the therapeutic is formulated with appropriate excipients into a pharmaceutical composition that, upon administration, releases the therapeutic in a controlled manner. Examples include single or multiple unit tablet or capsule compositions, oil solutions, suspensions, emulsions, microcapsules, microspheres, molecular complexes, nanoparticles, patches, and liposomes.

### Parenteral Compositions

The pharmaceutical composition may be administered parenterally by injection, infusion or implantation (subcutaneous, intravenous, intramuscular, intraperitoneal, intravesicularly or the like) in dosage forms, formulations, or via suitable delivery devices or implants containing conventional, non-toxic pharmaceutically acceptable carriers and adjuvants. The formulation and preparation of such compositions are well known to those skilled in the art of pharmaceutical formulation. Formulations can be found in Remington: The Science and Practice of Pharmacy, supra.

Compositions for parenteral use may be provided in unit dosage forms (e.g., in single-dose ampoules), or in vials containing several doses and in which a suitable preservative may be added (see below). The composition may be in the form of a solution, a suspension, an emulsion, an infusion device, or a delivery device for implantation, or it may be presented as a dry powder to be reconstituted with water or another suitable vehicle before use. Apart from the active agent that reduces or ameliorates a cancer, the composition may include suitable parenterally acceptable carriers and/or excipients. The active therapeutic agent(s), *e.g.* SEQ ID NOS: 1, 2 or the combination thereof, may be incorporated into microspheres, microcapsules, nanoparticles, liposomes, or the like for controlled release. Furthermore, the composition may include suspending, solubilizing, stabilizing, pH-adjusting agents, tonicity adjusting agents, and/or dispersing, agents.

As indicated above, the pharmaceutical compositions according to the invention may be in the form suitable for sterile injection. To prepare such a composition, the suitable therapeutic(s) are dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution, and isotonic sodium chloride solution and dextrose solution. The aqueous formulation may also contain one or more preservatives (e.g., methyl, ethyl or n-propyl p-hydroxybenzoate). In cases where one of the compounds is only sparingly or slightly soluble in water, a dissolution enhancing or solubilizing agent can be added, or the solvent may include 10-60% w/w of propylene glycol or the like.

### Controlled Release Parenteral Compositions

Controlled release parenteral compositions may be in form of aqueous suspensions, microspheres, microcapsules, magnetic microspheres, oil solutions, oil suspensions, or emulsions. Alternatively, the antibody may be incorporated in biocompatible carriers, liposomes, nanoparticles, implants, or infusion devices.

Materials for use in the preparation of microspheres and/or microcapsules are, e.g., biodegradable/bioerodible polymers such as polygalactin, poly-(isobutyl cyanoacrylate), poly(2-hydroxyethyl-L-glutam- nine) and, poly(lactic acid). Biocompatible carriers that may be used when formulating a controlled release parenteral formulation are carbohydrates (e.g., dextrans), proteins (e.g., albumin), lipoproteins, or antibodies. Materials for use in implants can be non-biodegradable (e.g., polydimethyl siloxane) or biodegradable (e.g., poly(caprolactone), poly(lactic acid), poly(glycolic acid) or poly(ortho esters) or combinations thereof).

### Solid Dosage Forms For Oral Use

Formulations for oral use include tablets containing the active ingredient(s) in a mixture with non-toxic pharmaceutically acceptable excipients. Such formulations are known to the skilled artisan. Excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, and antiadhesives (e.g., magnesium stearate, zinc stearate, stearic acid, silicas, hydrogenated vegetable oils, or talc). Other pharmaceutically acceptable excipients can be colorants, flavoring agents, plasticizers, humectants, buffering agents, and the like.

The tablets may be uncoated or they may be coated by known techniques, optionally to delay disintegration and absorption in the gastrointestinal tract and thereby providing a sustained action over a longer period. The coating may be adapted to release the active drug in a predetermined pattern (e.g., in order to achieve a controlled release formulation) or it may be adapted not to release the active drug until after passage of the stomach (enteric coating). The coating may be a sugar coating, a film coating (e.g., based on hydroxypropyl methylcellulose, methylcellulose, methyl hydroxyethylcellulose, hydroxypropylcellulose, carboxymethylcellulose, acrylate copolymers, polyethylene glycols and/or polyvinylpyrrolidone), or an enteric coating (e.g., based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose). Furthermore, a time delay material, such as, e.g., glyceryl monostearate or glyceryl distearate may be employed.

The solid tablet compositions may include a coating adapted to protect the composition from unwanted chemical changes, (e.g., chemical degradation prior to the release of the chimeric antibody). The coating may be applied on the solid dosage form in a similar manner as that described in Encyclopedia of Pharmaceutical Technology, supra.

Formulations for oral use may also be presented as chewable tablets, or as hard gelatin capsules wherein the active ingredient is mixed with an inert solid diluent (e.g., potato starch, lactose, microcrystalline cellulose, calcium carbonate, calcium phosphate or kaolin), or as soft gelatin capsules wherein the active ingredient is mixed with water or an oil medium, for example, peanut oil, liquid paraffin, or olive oil. Powders and granulates may be prepared using the ingredients mentioned above under tablets and capsules in a conventional manner using, e.g., a mixer, a fluid bed apparatus or a spray drying equipment.

### Controlled Release Oral Dosage Forms

Controlled release compositions for oral use may, e.g., be constructed to release the chimeric antibody therapeutic by controlling the dissolution and/or the diffusion of the active substance. Dissolution or diffusion controlled release can be achieved by appropriate coating of a tablet, capsule, pellet, or granulate formulation of compounds, or by incorporating the compound into an appropriate matrix. A controlled release coating may include one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3 butylene glycol, ethylene glycol methacrylate, and/or polyethylene glycols. In a controlled release matrix formulation, the matrix material may also include, e.g., hydrated metylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

A controlled release composition containing one or more therapeutic compounds may also be in the form of a buoyant tablet or capsule (i.e., a tablet or capsule that, upon oral administration, floats on top of the gastric content for a certain period of time). A buoyant tablet formulation of the compound(s) can be prepared by granulating a mixture of the compound(s) with excipients and 20-75% w/w of hydrocolloids, such as hydroxyethylcellulose, hydroxypropylcellulose, or hydroxypropylmethylcellulose. The obtained granules can then be compressed into tablets. On contact with the gastric juice, the tablet forms a substantially water-impermeable gel barrier around its surface. This gel barrier takes part in maintaining a density of less than one, thereby allowing the tablet to remain buoyant in the gastric juice.

### Kits

The invention provides kits for the treatment or prevention of neoplasia. In one embodiment, the kit includes a therapeutic or prophylactic composition containing a therapeutically effective amount of IL-2, e.g. SEQ ID NO: 1, in unit dosage form and one or more therapeutic agents. In some embodiments, the kit comprises a sterile container which contains a therapeutic or prophylactic cellular composition; such containers can be boxes, ampoules, bottles, vials, tubes, bags, pouches, blister-packs, or other suitable container forms known in the art. Such containers can be made of plastic, glass, laminated paper, metal foil, or other materials suitable for holding medicaments.

If desired IL-2, *e.g.* SEQ ID NO: 1, and one or more therapeutic agents are provided together with instructions for administering the IL-2, *e.g.* SEQ ID NO: 1, and one or more therapeutic agents to a subject in need of such therapy. The instructions will generally include information about the use of the composition for the treatment or prevention of the particular disease or disorder, *e.g.* an autoimmune disease. In other embodiments, the instructions include at least one of the following: description of the therapeutic agent; dosage schedule and administration for treatment or prevention of ischemia or symptoms thereof; precautions; warnings; indications; counter-indications; overdosage information; adverse reactions; animal pharmacology; clinical studies; and/or references. The instructions may be printed directly on the container (when present), or as a label applied to the container, or as a separate sheet, pamphlet, card, or folder supplied in or with the container.

The following examples further illustrates the invention. These examples are not intended to limit the invention in any manner.

### EXAMPLES

### EXAMPLE 1: Amino acid sequence of the aldesleukin obtained by the production method of the disclosure.

The amino acid sequence of the aldesleukin obtained by the production method of the disclosure was deduced by DNA sequencing of the aldesleukin gene present in the expression plasmid used in the method. FIG. 1 shows the obtained amino acid sequence.

### EXAMPLE 2: Specific activity of the aldesleukin/SDS aggregates obtained by the production method of the disclosure.

The biological activity of the aldesleukin/SDS aggregates obtained by the production method of the disclosure was determined by the proliferation of the HT-2 cell line assay. The specific activity of 20 independent lots was in the range of 16.6 - 19.5 IU/mg calibrated with the WHO International Standard for INTERLEUKIN 2 (Human, rDNA derived) NIBSC code: 86/500. This range of specific activity is similar to the declared specific activity of the aldesleukin/SDS aggregates prepared according to EP 1 688 146 B1.

### EXAMPLE 3: Comparative size, measured by dynamic light scattering, of the SDS/Proleukin aggregates obtained after reconstitution from the lyophilized commercial preparation and the SDS/aldesleukin aggregates obtained using the method of the disclosure.

A liquid preparation containing 2.2 mg/mL aldesleukin was obtained with the method of the invention with the following final composition: 0.44 mg/ml SDS, 50 mg/ml mannitol, 1.19 mg/ml disodium phosphate, 0.26 mg/ml monosodium di-hydrated phosphate, pH 7.5. This preparation was analyzed by dynamic light scattering to determine the size and polydispersity of the aldesleukin/SDS aggregates using a Zetasizer Nano ZS and the Zeta Nano Series software (Malvern Instruments, Germany). 100 µL sample was diluted in 1 mL pure water and measured at 25 °C five times for 30 sec with 30 sec equilibration time between measurements. Particle size is expressed as the hydrodynamic diameter in nm. FIG. 2 shows dynamic light scattering patterns for the SDS/aldesleukin aggregates obtained using the method of the disclosure and for PROLEUKIN^{™} after reconstitution from the lyophilized commercial preparation. The aldesleukin/SDS aggregates obtained by the production method of the disclosure display an extended size range between 4 and 18 nm with a peak at about 8 nm. PROLEUKIN^{™} also shows a similar distribution curve. However, the area under this curve is clearly lower than the area under the curve of the aldesleukin/SDS particles prepared by the method of the disclosure. Since the protein mass used in both analyzed samples was the same, this result suggests that upon reconstitution, part of the PROLEUKIN^{™} may remain as large aggregates not amenable to be studied with the used technique.

### EXAMPLE 4: Stability of the SDS/aldesleukin preparation of the aldesleukin obtained by the production method of the disclosure

The liquid preparation described in example 3 was tested for stability. Table 1 shows the activity changes in specific activity with the storage time at 2-8 °C. Besides the specific activity, in each sample the following characteristics were assayed: visual observation for anomalies, pH, volume, Western Blot, SDS PAGE (reducing and non-reducing conditions), Lowry, endotoxin contamination (LAL test) and sterility. At all measured times, these assays resulted normal.

**TABLE 1: Specific activity of the soluble aldesleukin/SDS aggregates obtained by the method of this disclosure**

| | | | | | |
|---|---|---|---|---|---|
| | | | | | |
| Time (months) | 0 | 3 | 6 | 9 | 12 |
| Specific activity (IU/mg) | 18.8 | 17.1 | 16.5 | 16.9 | 17.4 |

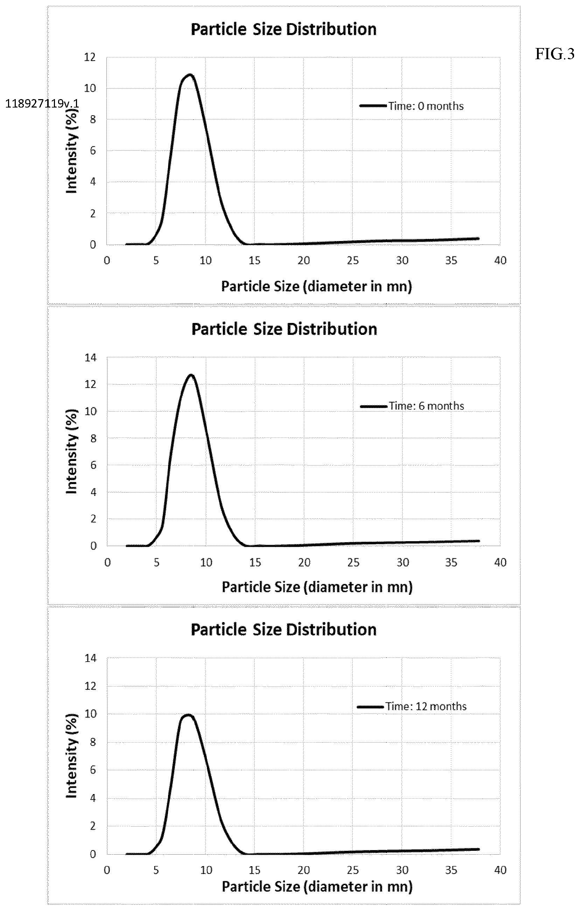

Furthermore, FIG. 3 shows that the size distribution of the aldesleukin/SDS aggregates, as measured by dynamic light scattering, is well preserved in the aldesleukin liquid composition of the disclosure after 6- or 12-month storage at 2-8 °C.

### EXAMPLE 5: Regulatory T cells (Treg) expansion in human peripheral mononuclear blood cells (PMBC) incubated with the SDS/aldesleukin aggregates obtained by the production method of the disclosure

Treg cells were recovered from human PMBC using the DYNABEADS^{™} Regulatory CD4⁺/CD25⁺ T Cell Kit (ThermoFisher Scientific). Aldesleukin/SDS aggregates capacity to induce Treg proliferation was evaluated using the CD3/CD28 DYNABEADS^{®} Human Treg Expander ((ThermoFisher Scientific) protocol. 500IU of the aldesleukin preparations were used for Treg expansion. After 7 days expansion Tregs were counted. Expansion was 76-125 times for both, the aldesleukin/SDS aggregates obtained using the method of the disclosure or the method fully disclosed in EP1 688 146 B1.

### EXAMPLE 6: Therapeutic activity of the aldesleukin/SDS aggregates obtained using the production method of the disclosure

A pulmonary metastasis model using B16F10 tumor cells as described (7), was used in order to compare the therapeutic efficiency of the aggregates of aldesleukin prepared by the method of this disclosure and the aldesleukin/SDS aggregates of PROLEUKIN^{™}. Eight-week old female C57BL/6 mice were intravenously injected into the tail vein with 0.5 ml of a cell suspension containing 2 × 10⁵ B16 cells. After this, mice were separated in three groups of ten mice each. Group 1 mice received a 7.0 mg/Kg dose of PROLEUKIN^{™}, daily from days 3 to 10. Group 2 mice received a 7.0 mg/Kg dose of the micro-aggregates of aldesleukin prepared by the method of this disclosure, daily from days 3 to 10. Group 3 mice received vehicle daily from days 3 to 10. Mice were euthanized on day 16 after tumor inoculation and lung metastases counted as described (7). Table 2 shows that the median number of metastases observed with the micro-aggregates prepared by the method of this disclosure was 4.3 (range 1-12), compared to 5.5 (range 3-22) with PROLEUKIN^{™}, being this difference not significant.

**TABLE 2: Therapeutic activity of the aldesleukin/SDS aggregates obtained using the production method of the disclosure**

| Treatment | metastases (mean) | range |
|---|---|---|
| Vehicle | 137.6 | 39-280 |
| PROLEUKIN^{™} | 5.5 | 3-22 |
| Aldesleukin (Aggregates of the disclosure) | 4.3 | 1-12 |

The aldesleukin prepared by the method of this disclosure and used in this assay was freshly produced. In contrast, Table 3 shows the same assay performed with the aldesleukin prepared by the method of this disclosure after storage for 6 or 12 months at 2-8 °C.

**TABLE 3: Therapeutic activity of the aldesleukin/SDS aggregates obtained using the production method of the disclosure after 6 or 12 months of storage at 2-8 °C for 6 or 12 months. These results reinforce the idea that the liquid preparation of aldesleukin obtained by the method of the disclosure, is stable for at least a year.**

| Treatment | metastases (mean) | range |
|---|---|---|
| Vehicle 6 month storage | 182 | 24-332 |
| Aldesleukin (Aggregates of the disclosure) 6 month storage | 8,4 | 0-35 |
| Vehicle 12 month storage | 123.2 | 12-156 |
| Aldesleukin (Aggregates of the disclosure) 12 month storage | 3.7 | 1-27 |

### SEQUENCES

### SEQ ID NO: 1

### LENGTH: 132

### PRT

ORGANISM: Artificial Sequence, mature des-alanyl-1 serine 125 variant of human interleukin-2

Pro Thr Ser Ser Ser Thr Lys Lys Thr Gln Leu Gln Leu Glu His Leu Leu Leu Asp Leu Gln
Met Ile Leu Asn Gly Ile Asn Asn Tyr Lys Asn Pro Lys Leu Thr Arg Met Leu Thr Phe Lys
Phe Tyr Met Pro Lys Lys Ala Thr Glu Leu Lys His Leu Gln Cys Leu Glu Glu Glu Leu Lys
Pro Leu Glu Glu Val Leu Asn Leu Ala Gln Ser Lys Asn Phe His Leu Arg Pro Arg Asp Leu

Ile Ser Asn Ile Asn Val Ile Val Leu Glu Leu Lys Gly Ser Glu Thr Thr Phe Met Cys Glu Tyr
Ala Asp Glu Thr Ala Thr Ile Val Glu Phe Leu Asn Arg Trp Ile Thr Phe Ser Gln Ser Ile Ile
Ser Thr Leu Thr

### References

### US PATENT DOCUMENTS

US 4,604,377 Aug. 5, 1986

### OTHER PATENT DOCUMENTS

EP 1 688 146 B1 18.07.2007
WO 2 017 068 031 A1 Abr. 27, 2017

### OTHER PUBLICATIONS

1- Malek TR. The biology of interleukin-2. Annu Rev Immunol. (2008). 26:453-5 79. DOI: 10.1146/annurev.immunol.26.021607.090357
2- Dudley ME et al. Adoptive Cell Transfer Therapy Following NonMyeloablative but Lymphodepleting Chemotherapy for the Treatment of Patients With Refractory Metastatic Melanoma. Journal of Clinical Oncology. (2005). 23: 2346-57. DOI: 10.1200/JCO.2005.00.240
3- Langerman A, Callender GG, Nishimura MI. Retroviral transduction of peptide stimulated t cells can generate dual t cell receptor-expressing (bifunctional) t cells reactive with two defined antigens. Journal of Translational Medicine. (2004). 2:4-8. DOI: 10.1186/1479-5876-2-42
4- Magee MS, Snook AE. Challenges to chimeric antigen receptor (CAR)-T cell therapy for cancer. Discov Med. (2014). 18:265-71
5- Arenas-Ramirez N, Woytschak J, Boyman O. Interleukin-2: Biology, Design and Application. Trends Immunol. (2015). 36:763-777. DOI: 10.1016/j.it.2015.10.003
6- Hank JA, Surfus J, Gan , Albertini M, Lindstrom M, Schiller JH, Hotton KM, Khorsand M, Sondel PM. Distinct clinical and laboratory activity of two recombinant interleukin-2 preparations. Clin Cancer Res. 1999 5:281-9
7- Overwijk WW, Restifo NP. B16 as a Mouse Model for Human Melanoma. Curr Protoc Immunol. (2001). CHAPTER: Unit-20.1.

### OTHER EMBODIMENTS

The invention is further illustrated by the following numbered clauses
1. A pharmaceutical composition comprising IL-2 in a range from about 0.001 mg to 3 mg/mL, sodium dodecyl sulfate (SDS) from about 0.0001 mg/ml to about 10 mg/mL, a non-ionic osmolytes(s), or combinations thereof.
2. The pharmaceutical composition of clause 1, wherein the IL-2 comprises SEQ ID NO: 1 or variants thereof.
3. The pharmaceutical composition of clause 1, wherein the osmolytes comprise mannitol, sorbitol, xylitol or a pH 7-8 phosphate buffer.
4. A method of inducing proliferation of regulatory T cells *in vivo,* comprising administering to a subject in need thereof, a therapeutically effective amount of aldesleukin wherein the regulatory T cells increase as compared to a control, inducing proliferation of regulatory T cells *in vivo.*
5. The method of clause 4, wherein the aldesleukin is comprised in a pharmaceutical composition.
6. The method of clause 4, wherein the aldesleukin comprises SEQ ID NO: 1 or variants thereof.
7. The method of clause 4, wherein the aldesleukin remains stable in the pharmaceutical liquid composition for at least a year as measured by an *in vitro* biological activity assay.
8. The method of clause 4, wherein the aldesleukin comprises a stable aldesleukin/SDS aggregate distribution in the liquid composition at least for a year in a range of about 4 - 18 nm with a peak at about 8 nm as measured by dynamic light scattering.
9. The method of clause 4, wherein the aldesleukin has a stable therapeutic activity, at least for a year in the liquid composition, as measured in an animal model of human cancer.
10. A method of treating an autoimmune disease or disorder in a subject in need thereof, comprising: administering to a subject in need thereof, a pharmaceutical composition comprising a therapeutically effective amount of aldesleukin wherein the regulatory T cells increase as compared to a control, inducing proliferation of regulatory T cells *in vivo.*
11. The method of clause 10, wherein the aldesleukin remains stable in the pharmaceutical liquid composition for at least a year as measured by an *in vitro* biological activity assay.
12. The method of clause 6, wherein the aldesleukin comprises a stable aldesleukin/SDS aggregate distribution in the liquid composition at least for a year in a range of about 4 - 18 nm with a peak at about 8 nm as measured by dynamic light scattering.
13. The method of clause 6, wherein the aldesleukin has a stable therapeutic activity, at least for a year in the liquid composition, as measured in an animal model of human cancer.
14. A method of regulating an immune response associated with a disease or disorder in a subject in need thereof, comprising: administering to a subject in need thereof, a pharmaceutical composition comprising a therapeutically effective amount of aldesleukin wherein the regulatory T cells increase as compared to a control, inducing proliferation of regulatory T cells *in vivo.*
15. The method of clause 10, wherein the aldesleukin remains stable in the pharmaceutical liquid composition for at least a year as measured by an *in vitro* biological activity assay.
16. The method of clause 10, wherein the aldesleukin comprises a stable aldesleukin/SDS aggregate distribution in the liquid composition at least for a year in a range of about 4 - 18 nm with a peak at about 8 nm as measured by dynamic light scattering.
17. The method of clause 10, wherein the aldesleukin has a stable therapeutic activity, at least for a year in the liquid composition, as measured in an animal model of human cancer.
18. The method of clause 10, wherein the disease or disorder comprises: an autoimmune disease, cancer, inflammation, a viral infection, a bacterial infection, a neurodegenerative disorder or combinations thereof.
19. A method of producing aldesleukin suitable for the preparation of a composition comprising aldesleukin in a pharmaceutical aqueous vehicle, the method comprising:
   fermenting a bacterial cell transfected with an expression vector encoding an aldesleukin gene;
   disrupting the bacterial cell;
   collecting inclusion bodies containing aldesleukin;
   subjecting the inclusion bodies to dissolution by a detergent followed by oxidation and a first chromatography;
   diluting with an organic nitrile and subjecting to a second chromatography;
   subjecting the aldesleukin to diafiltration and sterilization.
20. The method of clause 19, wherein the bacterial cell is an *Escherichia coli* bacterial cell.
21. The method of clause 19, wherein the detergent is sodium dodecyl sulfate (SDS).
22. The method of clause 19, wherein the oxidation is conducted with an oxidizing compound.
23. The method of clause19, wherein the first chromatography is a ceramic hydroxyapatite chromatography.
24. The method of clause 19, wherein the second chromatography is a high-performance liquid chromatography (HPLC).
25. The method of clause 24, wherein the HPLC is C4 column HPLC chromatography.
26. The method of clause 1, wherein the aldesleukin remains stable in the pharmaceutical liquid composition for at least a year as measured by an *in vitro* biological activity assay.
27. The method of clause 19, wherein the aldesleukin comprises a stable aldesleukin/SDS aggregate distribution in the liquid composition at least for a year in a range of about 4 - 18 nm with a peak at about 8 nm as measured by dynamic light scattering.
28. The method of clause 19, wherein the aldesleukin has a stable therapeutic activity, at least for a year in the liquid composition, as measured in an animal model of human cancer.
29. The method of clause 19, wherein the aldesleukin is used as a drug for medical treatments involving cells expressing IL-2 receptors in their membranes, comprising NK cells, T cells, dendritic cells, nonlymphoid cells, cell lines, transformed cells or combinations thereof.
30. A pharmaceutical composition comprising aldesleukin in which the aldesleukin: a) remains stable in the pharmaceutical liquid composition for at least a year as measured by the *in vitro* biological activity assay, b) have a stable aldesleukin/SDS aggregate distribution in the liquid composition at least for a year in the range of 4 - 18 nm range with a peak at about 8 nm as measure by dynamic light scattering, c) have a stable therapeutic activity, at least for a year in the liquid composition, as measure in an animal model of human cancer and d) is adequate to be used as a drug for treatments involving cells expressing IL-2 receptors in their membranes.
31. A method of restoring T-cell mediated immune responses in a subject in need thereof comprising administering a therapeutically effective amount of (i) a pharmaceutical composition comprising IL-2 in a range from about 0.001 mg to 3 mg/mL, sodium dodecyl sulfate (SDS) from about 0.0001 mg/ml to about 10 mg/mL, a non-ionic osmolytes(s), or combinations thereof (b) an expression vector encoding a therapeutic protein; (ii) one or more mobilization factors, thereby restoring T-cell mediated immune responses in the subject in need thereof.
32. The method of clause 31, wherein the IL-2 comprises SEQ ID NO: 1 or variants thereof.
33. The method of clause 31, wherein the osmolytes comprise mannitol, sorbitol, xylitol or a pH 7-8 phosphate buffer.
34. The method of clause 31, wherein the subject is suffering from an immune deficiency.
35. The method of clause 34, wherein the subject is suffering from Autoimmune Lymphoproliferative Syndrome (ALPS), APS-1 (APECED), CARD9, Chronic Granulomatous Disease (CGD), Congenital Neutropenia Syndromes, Common Variable Immunodeficiency (CVID), CTLA4 Deficiency, DOCK8 Deficiency, Glycosylation Disorders with Immunodeficiency, Hyper-Immunoglobulin E Syndromes (HIES), PI3 Kinase Disease, PLAID, Severe Combined Immunodeficiency (SCID), STAT3 Dominant-Negative Disease, WHIM Syndrome, X-Linked Agammaglobulinemia (XLA), X-Linked Lymphoproliferative Disease (XLP), Wiscott-Aldrich syndrome, DiGeorge syndrome, Ataxia-telangectasia, Chronic granulomatous disease, Transient hypogammaglobulinemia of infancy, Agammaglobulinemia, Complement deficiencies, or Selective IgA deficiency.
36. A method of treating a subject suffering from an immune deficiency disease comprising administering to the subject:
   (i) an expression vector encoding SEQ ID NO: 1; and/or,
   (ii) a cell comprising an expression vector encoding SEQ ID NO: 1; and/or,
   (iii) a pharmaceutical composition comprising IL-2 in a range from about 0.001 mg to 3 mg/mL, sodium dodecyl sulfate (SDS) from about 0.0001 mg/ml to about 10 mg/mL, a non-ionic osmolytes(s), or combinations thereof.
37. A method of treating a subject in need of immunotherapy, comprising: administering to the subject:
   (i) an expression vector encoding SEQ ID NO: 1; and/or,
   (ii) a cell comprising an expression vector encoding SEQ ID NO: 1,
      thereby treating the subject.
38. The method of clause 37, wherein the subject is suffering from cancer, immunodeficiency, immunosuppression, viral infections, an immune disorder or combinations thereof.
39. The method of clause 38, wherein the immune disorder, inflammation, graft versus host disease (GVHD), transplant rejection, or an autoimmune disorder.
40. The method of clause 39, wherein the immune disorder is multiple sclerosis, inflammatory bowel disease, rheumatoid arthritis, type I diabetes, systemic lupus erythrematosus, contact hypersensitivity, asthma or Sjogren's syndrome.
41. The method of clause 37, further comprising administering IL-2.
42. The method of clause 37, wherein the cells comprise: autologous cells, allogeneic cells, haplotype matched cells, haplotype mismatched cells, haplo-identical cells, xenogeneic cells, cell lines or combinations thereof.
43. The method of claim 37, wherein the cells comprise stem cells, cord blood cells, adult stem cells, mesenchymal stem cells, mesenchymal stromal cells, induced pluripotent stem cells, autologous stem cells, bone marrow cells, hematopoietic cells, hematopoietic stem cells, somatic cells, germ line cells, differentiated cells, somatic stem cells, embryonic stem cells or combinations thereof

From the foregoing description, it will be apparent that variations and modifications may be made to the invention described herein to adopt it to various usages and conditions. Such embodiments are also within the scope of the following claims.

All citations to sequences, patents and publications in this specification are herein incorporated by reference to the same extent as if each independent patent and publication was specifically and individually indicated to be incorporated by reference.

## Claims

1. A pharmaceutical composition comprising IL-2 in a range from about 0.001 mg to 3 mg/mL, sodium dodecyl sulfate (SDS) from about 0.0001 mg/ml to about 10 mg/mL, a non-ionic osmolytes(s), or combinations thereof.

2. The pharmaceutical composition of claim 1, wherein the IL-2 comprises SEQ ID NO: 1 or variants thereof.

3. The pharmaceutical composition of claim 1 or claim 2, wherein the osmolytes comprise mannitol, sorbitol, xylitol or a pH 7-8 phosphate buffer.

4. Aldesleukin for use in a method of inducing proliferation of regulatory T cells *in vivo,* comprising administering to a subject in need thereof, a therapeutically effective amount of the aldesleukin wherein the regulatory T cells increase as compared to a control, inducing proliferation of regulatory T cells *in vivo.*

5. The aldesleukin for use of claim 4, wherein the aldesleukin is comprised in a pharmaceutical composition; or
wherein the aldesleukin comprises SEQ ID NO: 1 or variants thereof.

6. A pharmaceutical composition comprising a therapeutically effective amount of aldesleukin, for use in treating an autoimmune disease or disorder, wherein the regulatory T cells increase as compared to a control, inducing proliferation of regulatory T cells *in vivo.*

7. A pharmaceutical composition comprising aldesleukin for use in regulating an immune response associated with a disease or disorder in a subject in need thereof, comprising: administering to a subject in need thereof, the pharmaceutical composition comprising a therapeutically effective amount of aldesleukin, wherein the regulatory T cells increase as compared to a control, inducing proliferation of regulatory T cells *in vivo.*

8. The aldesleukin for use of claim 4 or 5 or the pharmaceutical composition for use of claim 6 or 7, wherein the aldesleukin is in the form of a pharmaceutical liquid composition and wherein the aldesleukin remains stable in the pharmaceutical liquid composition for at least a year as measured by an *in vitro* biological activity assay; and/or
wherein the aldesleukin comprises a stable aldesleukin/SDS aggregate distribution in the liquid composition at least for a year in a range of about 4 - 18 nm with a peak at about 8 nm as measured by dynamic light scattering; and/or
wherein the aldesleukin has a stable therapeutic activity, at least for a year in the liquid composition, as measured in an animal model of human cancer.

9. The pharmaceutical composition for use of claim 6 or claim 7, wherein the disease or disorder comprises: an autoimmune disease, cancer, inflammation, a viral infection, a bacterial infection, a neurodegenerative disorder or combinations thereof.

10. Aldesleukin for use in a treatment involving cells expressing IL-2 receptors in their membranes, comprising NK cells, T cells, dendritic cells, nonlymphoid cells, cell lines, transformed cells or combinations thereof; wherein the aldesleukin is produced by a method suitable for the preparation of a composition comprising aldesleukin in a pharmaceutical aqueous vehicle, the method comprising:
fermenting a bacterial cell transfected with an expression vector encoding an aldesleukin gene;
disrupting the bacterial cell;
collecting inclusion bodies containing aldesleukin;
subjecting the inclusion bodies to dissolution by a detergent followed by oxidation and a first chromatography;
diluting with an organic nitrile and subjecting to a second chromatography;
subjecting the aldesleukin to diafiltration and sterilization.

11. A pharmaceutical liquid composition comprising aldesleukin in which the aldesleukin:
a) remains stable in the pharmaceutical liquid composition for at least a year as measured by the *in vitro* biological activity assay, and/or
b) have a stable aldesleukin/SDS aggregate distribution in the liquid composition at least for a year in the range of 4 - 18 nm range with a peak at about 8 nm as measure by dynamic light scattering, and/or
c) have a stable therapeutic activity, at least for a year in the liquid composition, as measure in an animal model of human cancer, and/or
d) is adequate to be used as a drug for treatments involving cells expressing IL-2 receptors in their membranes.

12. A pharmaceutical composition according to any of claims 1-3, for use in a method of restoring T-cell mediated immune responses in a subject in need thereof, comprising administering a therapeutically effective amount of the pharmaceutical composition, an expression vector encoding a therapeutic protein, and optionally one or mobilization factors, thereby restoring T-cell mediated immune responses in the subject in need thereof.

13. The pharmaceutical composition for use of claim 12, wherein the subject is suffering from an immune deficiency; optionally
wherein the subject is suffering from Autoimmune Lymphoproliferative Syndrome (ALPS), APS-1 (APECED), CARD9, Chronic Granulomatous Disease (CGD), Congenital Neutropenia Syndromes, Common Variable Immunodeficiency (CVID), CTLA4 Deficiency, DOCK8 Deficiency, Glycosylation Disorders with Immunodeficiency, Hyper-Immunoglobulin E Syndromes (HIES), PI3 Kinase Disease, PLAID, Severe Combined Immunodeficiency (SCID), STAT3 Dominant-Negative Disease, WHIM Syndrome, X-Linked Agammaglobulinemia (XLA), X-Linked Lymphoproliferative Disease (XLP), Wiscott-Aldrich syndrome, DiGeorge syndrome, Ataxia-telangectasia, Chronic granulomatous disease, Transient hypogammaglobulinemia of infancy, Agammaglobulinemia, Complement deficiencies, or Selective IgA deficiency.

14. An expression vector encoding SEQ ID NO: 1; and/or,
a cell comprising an expression vector encoding SEQ ID NO: 1; and/or,
a pharmaceutical composition according to claims 1-3;
for use in treating a subject suffering from an immune deficiency disease.

15. An expression vector encoding SEQ ID NO: 1; and/or,
a cell comprising an expression vector encoding SEQ ID NO: 1,
for use in treating a subject in need of immunotherapy..

16. The expression vector or cell for use of claim 15, wherein the subject is suffering from cancer, immunodeficiency, immunosuppression, viral infections, an immune disorder or combinations thereof; optionally
wherein the immune disorder is selected from inflammation, graft versus host disease (GVHD), transplant rejection, or an autoimmune disorder; further optionally
wherein the immune disorder is multiple sclerosis, inflammatory bowel disease, rheumatoid arthritis, type I diabetes, systemic lupus erythrematosus, contact hypersensitivity, asthma or Sjogren's syndrome.

17. The expression vector or cell for use of claim 15 or claim 16, further comprising administering IL-2.

18. The cell for use of claims 15-17, wherein the cells comprise: autologous cells, allogeneic cells, haplotype matched cells, haplotype mismatched cells, haplo-identical cells, xenogeneic cells, cell lines or combinations thereof; or
wherein the cells comprise stem cells, cord blood cells, adult stem cells, mesenchymal stem cells, mesenchymal stromal cells, induced pluripotent stem cells, autologous stem cells, bone marrow cells, hematopoietic cells, hematopoietic stem cells, somatic cells, germ line cells, differentiated cells, somatic stem cells, embryonic stem cells or combinations thereof.
